# EUROPEAN PATENT APPLICATION

(11) **EP 4 737 441 A1**
(43) Date of publication of application: **06.05.2026**
(21) Application number: 24831653.1
(22) Date of filing: 11.06.2024
(51) Int. Cl.: C07D 209/86, C07D 471/04, C07F 5/02, C07F 7/08, C07F 7/10

(54) **METHOD FOR PRODUCING ELECTROPHILIC SUBSTITUTED AROMATIC COMPOUND**

(30) Priority: 28.06.2023 JP 2023106114
(71) Applicant: FUJIFILM Corporation, Tokyo 106-8620 (JP); FUJIFILM Wako Pure Chemical Corporation, Osaka 540-8605 (JP)
(72) Inventor: TSUNA, Kazuhiro, Ashigarakami-gun, Kanagawa 258-8577 (JP); IWASAKI, Koichi, Ashigarakami-gun, Kanagawa 258-8577 (JP); WADA, Kenji, Ashigarakami-gun, Kanagawa 258-8577 (JP)
(74) Representative: HGF
(86) International application number: PCT/JP2024/021147
(87) International publication number: WO 2025/004786

(57) **Abstract**

Disclosed is a method for manufacturing an electrophilic substituted aromatic compound, including continuously carrying out, by a flow-type reaction, a reaction in which a halogenated aromatic compound is reacted with an organometallic compound to produce a metallated aromatic compound, and the metallated aromatic compound is reacted with an electrophilic compound to produce an electrophilic substituted aromatic compound,
in which the method includes allowing a liquid containing the halogenated aromatic compound in an organic solvent, a liquid containing the organometallic compound in an organic solvent, and a liquid containing the electrophilic compound in an organic solvent to flow in a flow channel, with each liquid having a moisture content of 200 ppm or less.

## Description

### BACKGROUND OF THE INVENTION

### 1. Field of the Invention

The present invention relates to a method for manufacturing an electrophilic substituted aromatic compound.

### 2. Description of the Related Art

A substituted aromatic compound is used in a variety of applications as a physiologically active substance, a functional material, and the like, and as a synthetic intermediate thereof. As a method for synthesizing a substituted aromatic compound, a synthesis route is known in which an organometallic compound is reacted with a halogenated aromatic compound (halogeno-aromatic compound) to produce a metallated aromatic compound, and this metallated aromatic compound is then reacted with an electrophilic compound to produce an electrophilic substituted aromatic compound (hereinafter, also referred to as a "metallation/electrophilic substitution reaction of a halogenated aromatic compound"). The obtained electrophilic substituted aromatic compound is treated with a quenching agent such as water or alcohol, as necessary, or subjected to a desired chemical treatment, washing treatment, purification treatment, or the like to obtain a desired electrophilic substituted aromatic compound. For example, an organolithium compound such as butyl lithium as the organometallic compound is reacted with a brominated aromatic compound as the halogenated aromatic compound to produce a lithiated aromatic compound, and this lithiated aromatic compound is then reacted with an electrophilic compound to introduce a desired substituent into a lithiation site, whereby a desired electrophilic substituted aromatic compound can be obtained.

JP2021-8436A describes that, for the purpose of carrying out the manufacture of a lithiated aromatic compound in a higher yield, an organolithium compound and a reaction solvent species are mixed in advance before a reaction between the organolithium compound and a halogenated aromatic compound. In this manner, it is said that the heat generation due to mixing of the organolithium compound and the reaction solvent species during the lithiation reaction can be suppressed, and the production of by-products in the lithiation reaction can be suppressed. The obtained lithiated aromatic compound can be reacted with an electrophilic compound to obtain an electrophilic substituted lithium compound. JP2021-8436A also describes that the above reaction is carried out by a flow-type reaction.

In addition, JP2008-195639A describes that, for the purpose of efficiently manufacturing a desired o-disubstituted aromatic compound in a high yield and with a high selectivity using an o-dihalo aromatic compound as a raw material, a lithiation reaction of a halogenated benzene compound and a reaction of the obtained lithiation product with an electrophilic compound are carried out using a microreactor (flow-type reaction). According to the technique described in JP2008-195639A, it is said that the by-production of benzyne in the reaction product can be suppressed even in a case where the reaction is carried out at a relatively high temperature of -80°C to -50°C.

### SUMMARY OF THE INVENTION

In the metallation/electrophilic substitution reaction of a halogenated aromatic compound, it is common to use an excess amount of the electrophilic compound in order to promote the reaction and increase the production rate of the desired compound (the proportion of the molar amount of the desired electrophilic substituted aromatic compound to the molar amount of the halogenated aromatic compound, %). On the other hand, in view of recent social demands based on the SDGs and the like, it has been more strongly required to obtain a desired compound with higher efficiency while reducing waste of raw materials or reagents even in chemical synthesis reactions. In addition, in a case where an excessive amount of the electrophilic compound is used, there is also a problem of increased manufacturing costs, particularly in a case where the electrophilic compound is expensive.

An object of the present invention is to provide a method for manufacturing an electrophilic substituted aromatic compound through a metallation/electrophilic substitution reaction of a halogenated aromatic compound, which method makes it possible to obtain a desired electrophilic substituted aromatic compound with a sufficiently high production rate while further reducing the amount of an electrophilic compound used.

As a result of extensive studies in view of the above-mentioned problems, the present inventors have found that, in a case where the metallation/electrophilic substitution reaction of a halogenated aromatic compound is continuously carried out by a flow-type reaction, it is possible to obtain a desired electrophilic substituted aromatic compound at a higher production rate while reducing an amount of an electrophilic compound used by reducing a moisture content of each of a liquid containing an organometallic compound in an organic solvent, a liquid containing a halogenated aromatic compound in an organic solvent, and a liquid containing the electrophilic compound in an organic solvent to 200 ppm or less and allowing the liquids to flow in a flow channel. The present invention has been completed through further studies on the basis of these findings.

That is, the object of the present invention has been achieved by the following means.
[1] A method for manufacturing an electrophilic substituted aromatic compound, comprising: continuously carrying out, by a flow-type reaction, a reaction in which a halogenated aromatic compound is reacted with an organometallic compound to produce a metallated aromatic compound, and the metallated aromatic compound is reacted with an electrophilic compound to produce an electrophilic substituted aromatic compound,
   in which the method includes allowing a liquid containing the halogenated aromatic compound in an organic solvent, a liquid containing the organometallic compound in an organic solvent, and a liquid containing the electrophilic compound in an organic solvent to flow in a flow channel, with each liquid having a moisture content of 200 ppm or less.
[2] The method for manufacturing an electrophilic substituted aromatic compound according to [1], in which, in a case where the flow-type reaction is a one-stage reaction system, the electrophilic compound is used in an amount of 0.80 molar equivalents or more and less than 1.20 molar equivalents with respect to the halogenated aromatic compound, and
   in a case where the flow-type reaction is a multi-stage reaction system, the electrophilic compound is supplied to a final stage reaction in an amount of 0.80 molar equivalents or more and less than 2.00 molar equivalents with respect to the halogenated aromatic compound.
[3] The method for manufacturing an electrophilic substituted aromatic compound according to [1], in which, in a case where the flow-type reaction is a one-stage reaction system, the electrophilic compound is used in an amount of 0.85 molar equivalents or more and 1.05 molar equivalents or less with respect to the halogenated aromatic compound, and
   in a case where the flow-type reaction is a multi-stage reaction system, the electrophilic compound is supplied to a final stage reaction in an amount of 0.80 molar equivalents or more and 1.50 molar equivalents or less with respect to the halogenated aromatic compound.
[4] The method for manufacturing an electrophilic substituted aromatic compound according to [1], in which the flow-type reaction is a multi-stage reaction system, and the electrophilic compound is supplied to a final stage reaction in an amount of 0.80 molar equivalents or more and 1.20 molar equivalents or less with respect to the halogenated aromatic compound.
[5] The method for manufacturing an electrophilic substituted aromatic compound according to any one of [1] to [4], further comprising: preparing a liquid A1 containing the halogenated aromatic compound in an organic solvent, a liquid B1 and a liquid B2 each containing the organometallic compound in an organic solvent, and a liquid C1 and a liquid C2 each containing the electrophilic compound in an organic solvent; introducing the liquids A1, B1, B2, C1, and C2 into different flow channels to allow each liquid to flow in each flow channel; and allowing the liquid A1 and the liquid B1 to join together to form a joined liquid M1, in which a metallated halogeno-aromatic compound is produced in the joined liquid M1 while the joined liquid M1 flows downstream in a reaction flow channel F1; allowing the joined liquid M1 and the liquid C1 to join together to form a joined liquid M2, in which an electrophilic monosubstituted halogeno-aromatic compound is produced in the joined liquid M2 while the joined liquid M2 flows downstream in a reaction flow channel F2; allowing the joined liquid M2 and the liquid B2 to join together to form a joined liquid M3, in which a metallated electrophilic monosubstituted aromatic compound is produced in the joined liquid M3 while the joined liquid M3 flows downstream in a reaction flow channel F3; and allowing the joined liquid M3 and the liquid C2 to join together to form a joined liquid M4, in which an electrophilic disubstituted aromatic compound is produced in the joined liquid M4 while the joined liquid M4 flows downstream in a reaction flow channel F4.
[6] The method for manufacturing an electrophilic substituted aromatic compound according to any one of [1] to [3], further comprising: preparing a liquid A2 containing the halogenated aromatic compound in an organic solvent, a liquid B3 containing the organometallic compound in an organic solvent, and a liquid C3 containing the electrophilic compound in an organic solvent; introducing the liquids A2, B3, and C3 into different flow channels to allow each liquid to flow in each flow channel; and allowing the liquid A2 and the liquid B3 to join together to form a joined liquid M6, in which a metallated aromatic compound is produced in the joined liquid M6 while the joined liquid M6 flows downstream in a reaction flow channel F6; and allowing the joined liquid M6 and the liquid C3 to join together to form a joined liquid M7, in which an electrophilic monosubstituted aromatic compound is produced in the joined liquid M7 while the joined liquid M7 flows downstream in a reaction flow channel F7.
[7] The method for manufacturing an electrophilic substituted aromatic compound according to any one of [1] to [4], further comprising: preparing a liquid A3 and a liquid A4 each containing the halogenated aromatic compound in an organic solvent, a liquid B4 and a liquid B5 each containing the organometallic compound in an organic solvent, and a liquid C4 containing the electrophilic compound in an organic solvent; introducing the liquids A3, A4, B4, B5, and C4 into different flow channels to allow each liquid to flow in each flow channel; and allowing the liquid A3 and the liquid B4 to join together to form a joined liquid M9, in which a metallated aromatic compound is produced in the joined liquid M9 while the joined liquid M9 flows downstream in a reaction flow channel F9; allowing the liquid A4 and the liquid B5 to join together to form a joined liquid M11, in which a metallated aromatic compound is produced in the joined liquid M11 while the joined liquid M11 flows downstream in a reaction flow channel F11; allowing the joined liquid M9 and the liquid C4 to join together to form a joined liquid M10, in which an electrophilic monosubstituted aromatic compound (i) functioning as an electrophilic compound is produced in the joined liquid M10 while the joined liquid M10 flows downstream in a reaction flow channel F10; and allowing the joined liquid M11 and the joined liquid M10 to join together to form a joined liquid M12, in which the metallated aromatic compound in the joined liquid M11 reacts with the electrophilic monosubstituted aromatic compound (i) in the joined liquid M10 to produce an electrophilic monosubstituted aromatic compound in the joined liquid M12 while the joined liquid M12 flows downstream in a reaction flow channel F12.
[8] The method for manufacturing an electrophilic substituted aromatic compound according to any one of [1] to [4], further comprising: preparing a liquid A5 containing the halogenated aromatic compound in an organic solvent, a liquid B6 containing the organometallic compound in an organic solvent, a liquid C5 containing the electrophilic compound in an organic solvent, and a liquid D containing a nucleophilic compound in an organic solvent; introducing the liquids A5, B6, C5, and D into different flow channels to allow each liquid to flow in each flow channel; and allowing the liquid A5 and the liquid B6 to join together to form a joined liquid M14, in which a metallated aromatic compound is produced in the joined liquid M14 while the joined liquid M14 flows downstream in a reaction flow channel F14; allowing the joined liquid M14 and the liquid C5 to join together to form a joined liquid M15, in which an electrophilic monosubstituted aromatic compound is produced in the joined liquid M15 while the joined liquid M15 flows downstream in a reaction flow channel F15; and allowing the joined liquid M15 and the liquid D to join together to form a joined liquid M16, in which an electrophilic monosubstituted/nucleophilic substituted aromatic compound is produced in the joined liquid M16 while the joined liquid M16 flows downstream in a reaction flow channel F16.
[9] The method for manufacturing an electrophilic substituted aromatic compound according to any one of [1] to [8], in which all of moisture contents of the liquid containing the halogenated aromatic compound in an organic solvent, the liquid containing the organometallic compound in an organic solvent, and the liquid containing the electrophilic compound in an organic solvent, each of which is allowed to flow in the flow channel, are controlled to be 100 ppm or less.

In the present invention and the present specification, any numerical range expressed using "to" refers to a range that includes the numerical values before and after the "to" as a lower limit value and an upper limit value, respectively.

In the present invention and the present specification, in a case where the size of inner cross section of a tube (equivalent diameter) of a flow channel, a joining portion, a mixer, or the like is described, the size does not include a connecting portion between flow channels, a connecting portion between a flow channel and a joining portion, and a connecting portion between a flow channel and a mixer. That is, the size of each of the connecting portions is appropriately adjusted using a connecting tube or the like such that a fluid flows in the connecting portion from upstream to downstream.

In the present invention and the present specification, the terms "upstream" and "downstream" are used with respect to a direction where a liquid flows, and a side where the liquid is introduced (a side where the liquid flows in) is upstream, and a side where the liquid flows out is downstream.

In the present invention and the present specification, "ppm" is based on mass.

According to the method for manufacturing an electrophilic substituted aromatic compound according to the embodiment of the present invention, in the metallation/electrophilic substitution reaction of a halogenated aromatic compound, it is possible to obtain a desired electrophilic substituted aromatic compound with a sufficiently high production rate while reducing the amount of the electrophilic compound used.

### BRIEF DESCRIPTION OF THE DRAWINGS

FIG. 1 is a view for schematically illustrating one embodiment of a flow-type reaction system of the present invention (two-stage reaction system).
FIG. 2 is a reaction scheme of an example of a two-stage reaction system that is applicable to the flow-type reaction system of FIG. 1.
FIG. 3 shows the reaction scheme shown in FIG. 2 in more detail.
FIG. 4 is a view for schematically illustrating another embodiment of the flow-type reaction system of the present invention (one-stage reaction system).
FIG. 5 is a view for schematically illustrating still another embodiment of the flow-type reaction system of the present invention (one-stage reaction system).
FIG. 6 is a view for schematically illustrating still yet another embodiment of the flow-type reaction system of the present invention (one-stage reaction system).
FIG. 7 is a reaction scheme of an example of a one-stage reaction system that is applicable to the flow-type reaction system of FIG. 6.
FIG. 8 is a reaction scheme of another example of the two-stage reaction system.
FIG. 9 is a reaction scheme of another example of the one-stage reaction system.

### DESCRIPTION OF THE PREFERRED EMBODIMENTS

### [Manufacture of electrophilic substituted aromatic compound by flow-type reaction]

The method for manufacturing an electrophilic substituted aromatic compound according to the embodiment of the present invention (hereinafter, also referred to as "the manufacturing method according to the embodiment of the present invention") employs a flow-type reaction. That is, the manufacturing method according to the embodiment of the present invention involves continuously carrying out, by a flow-type reaction, at least a reaction of reacting an organometallic compound with a halogenated aromatic compound to produce a metallated aromatic compound and a reaction of reacting the metallated aromatic compound with an electrophilic compound to produce an electrophilic substituted aromatic compound (metallation/electrophilic substitution reaction of a halogenated aromatic compound). The desired electrophilic substituted aromatic compound can be obtained in a desired form through a quenching treatment, a chemical treatment (oxidization treatment or the like), a washing treatment, a purification treatment, and the like, as necessary.

With regard to the manufacturing method according to the embodiment of the present invention, in the flow-type reaction, a liquid (A-I) containing the above-mentioned halogenated aromatic compound in an organic solvent, a liquid (B-I) containing the above-mentioned organometallic compound in an organic solvent, and a liquid (C-I) containing the above-mentioned electrophilic compound in an organic solvent are allowed to flow in different flow channels, respectively, and the respective flow channels are appropriately allowed to join together, thereby causing the above-mentioned reactions to occur. Usually, the liquid (A-I) and the liquid (B-I) are allowed to join together, and the joined liquid is then allowed to join together with the liquid (C-I). In the manufacturing method according to the embodiment of the present invention, the moisture content of each of the above-mentioned liquids flowing in each of the above-mentioned flow channels is controlled to be 200 ppm or less. By reducing the moisture content of each liquid to 200 ppm or less, it is possible to obtain the desired electrophilic substituted aromatic compound with a sufficiently high production rate even in a case where the amount of the electrophilic compound used is reduced (for example, even in a case where the amount of the electrophilic compound used is the minimum amount necessary based on the stoichiometric ratio). All of the liquids (A-I), (B-I), and (C-I) are usually solutions.

In the present invention, "allowing the liquid to flow in the flow channel with the moisture content reduced to 200 ppm or less" includes both of a form in which a liquid with a moisture content reduced to 200 ppm or less is introduced into the flow channel and a form in which a liquid with a moisture content of more than 200 ppm is introduced into the flow channel and passed through a dehydration device (a dehydration column or the like) disposed in the flow channel before joining with other liquids (that is, before the reaction occurs) to reduce the moisture content to 200 ppm. Various desiccants can be applied to the dehydration device. For example, a dehydration column packed with crystalline zeolite such as molecular sieve can be used as the dehydration device. The moisture content of each of the liquids (the liquids (A-I), (B-I), and (C-I)) flowing in the flow channel is preferably 170 ppm or less, preferably 150 ppm or less, more preferably 130 ppm or less, even still more preferably 120 ppm or less, even still further preferably 110 ppm or less, and even still more further preferably 100 ppm or less. In addition, the moisture content is also preferably 90 ppm or less, is also preferably 80 ppm or less, is also preferably 70 ppm or less, or is also preferably 60 ppm or less. The moisture content of each of the liquids flowing in the flow channel is practically 1 ppm or more, and is usually 3 ppm or more and preferably 5 ppm or more. Therefore, in a case where the moisture content of each of the liquids flowing in the flow channel is shown as the preferred range, it is preferably 1 to 200 ppm, more preferably 1 to 170 ppm, still more preferably 1 to 150 ppm, even still more preferably 3 to 130 ppm, even still further preferably 3 to 120 ppm, even still more further preferably 3 to 110 ppm, and even still yet more preferably 3 to 100 ppm. In addition, the moisture content range may be 5 to 90 ppm, may be 5 to 80 ppm, may be 5 to 70 ppm, or may be 5 to 60 ppm.

The above-mentioned flow-type reaction may be a reaction in which an electrophilic substituted aromatic compound obtained from a halogenated aromatic compound is an electrophilic monosubstituted aromatic compound (this flow-type reaction is referred to as "flow-type reaction is a one-stage reaction system" in the present invention), or a reaction in which an electrophilic substituted aromatic compound obtained from a halogenated aromatic compound is an electrophilic disubstituted aromatic compound (this flow-type reaction is referred to as "flow-type reaction is a two-stage reaction system" in the present invention). In addition, the above-mentioned flow-type reaction may be a reaction in which one molecule of an electrophilic substituted aromatic compound obtained from a halogenated aromatic compound is substituted with three or more electrophilic compounds (this flow-type reaction is referred to as "a reaction system in which the flow-type reaction is a reaction system of three or more stages" in the present invention). The above-mentioned two-stage reaction system and three-stage or more reaction system are collectively referred to as "the flow-type reaction is a multi-stage reaction system". The above-mentioned flow-type reaction is preferably a one- to three-stage reaction system and more preferably a one-stage or two-stage reaction system.

For example, in the metallation/electrophilic substitution reaction of a halogenated aromatic compound, in a case where one molecule of the halogenated aromatic compound has only one halogen element to be substituted with a metal, the halogenated aromatic compound is reacted with an organometallic compound, and the resulting monometallated aromatic compound is reacted with an electrophilic compound to produce a desired electrophilic monosubstituted aromatic compound, which is therefore classified as a one-stage reaction system in the present invention.

In addition, for example, in the metallation/electrophilic substitution reaction of a halogenated aromatic compound, in a case where one molecule of the halogenated aromatic compound has two halogen elements to be substituted with a metal, the halogenated aromatic compound is first reacted with an organometallic compound, and the resulting metallated halogeno-aromatic compound is then reacted with an electrophilic compound to produce an electrophilic monosubstituted halogeno-aromatic compound (first stage reaction). Next, the desired electrophilic disubstituted aromatic compound can be obtained through a step of reacting the electrophilic monosubstituted halogeno-aromatic compound with an organometallic compound, and reacting the resulting metallated electrophilic monosubstituted aromatic compound with an electrophilic compound to produce an electrophilic disubstituted aromatic compound (second stage reaction). In this case, the reaction system is classified as a two-stage reaction system in the present invention.

An embodiment of the flow-type reaction system used in the present invention will be described with reference to the accompanying drawings. It should be noted that each of the drawings is an explanatory view for facilitating understanding of the present invention, and the sizes or relative size relationships of the respective members may be changed for the sake of convenience of explanation and do not represent the actual relationships. In addition, external shapes or shapes other than those specified in the present invention are not limited to those shown in the drawings.

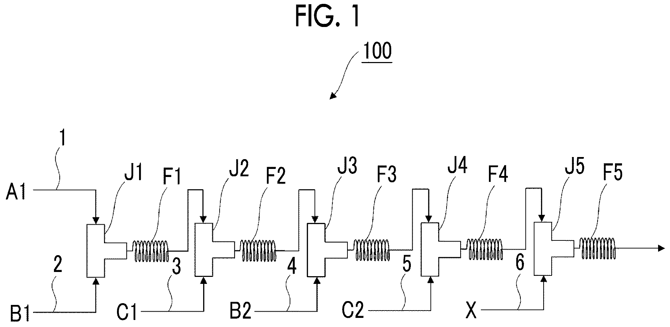

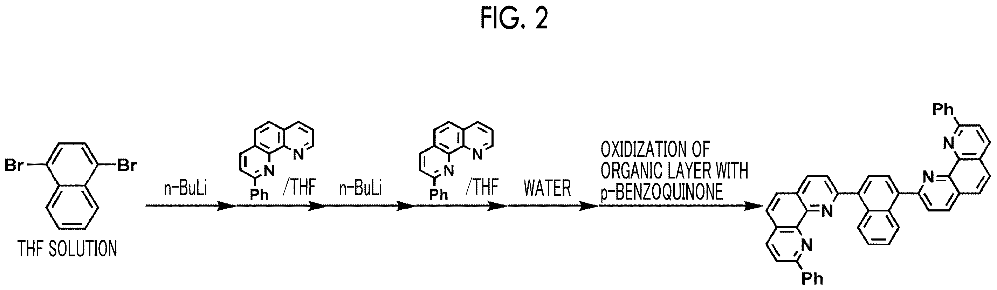

FIG. 1 is a schematic view showing an example of the flow-type reaction system used in the manufacturing method according to the embodiment of the present invention. In addition, FIG. 2 is a reaction scheme showing an example of a metallation/electrophilic substitution reaction of a halogenated aromatic compound that is applied to the flow-type reaction system of FIG. 1. 1,4-dibromonaphthalene is used as the halogenated aromatic compound, n-butyl lithium (n-BuLi) is used as the organometallic compound, and 2-phenyl-1,10-phenanthroline is used as the electrophilic compound.

A flow-type reaction system 100 shown in FIG. 1 is a two-stage reaction system in which a liquid B1 and a liquid B2 each containing an organometallic compound in an organic solvent and a liquid C1 and a liquid C2 each containing an electrophilic compound in an organic solvent are each introduced in two stages into a liquid A1 containing a halogenated aromatic compound (for example, a dihalogenated aromatic compound) in an organic solvent. The organometallic compound contained in the liquid B1 and the organometallic compound contained in the liquid B2 may be the same as or different from each other. In addition, the electrophilic compound contained in the liquid C1 and the electrophilic compound contained in the liquid C2 may be the same as or different from each other depending on the chemical structure of the desired electrophilic disubstituted aromatic compound. This also applies to the description of other embodiments.

In the flow-type reaction system 100, a flow channel 1 into which the liquid A1 is introduced, a flow channel 2 into which the liquid B1 is introduced, a joining portion J1 at which the flow channel 1 and the flow channel 2 join together, a reaction tube F1 connected to the downstream end part of the joining portion J1, a flow channel 3 into which the liquid C1 is introduced, a joining portion J2 at which the reaction tube F1 and the flow channel 3 join together, and a reaction tube F2 connected to the downstream end part of the joining portion J2 are portions related to the first stage reaction. In the first stage reaction, first, a metallated halogeno-aromatic compound is produced in a joined liquid M1 which is produced by the joining of the liquid A1 and the liquid B1 at the joining portion J1 while the joined liquid M1 flows in the reaction tube F1, and then, an electrophilic monosubstituted halogeno-aromatic compound is produced in a joined liquid M2 which is produced by the joining of the joined liquid M1 and the liquid C1 at the joining portion J2 while the joined liquid M2 flows in the reaction tube F2.

In addition, in the flow-type reaction system 100 shown in FIG. 1, a flow channel 4 into which the liquid B2 is introduced, a joining portion J3 at which the reaction tube F2 and the flow channel 4 join together, a reaction tube F3 connected to the downstream end part of the joining portion J3, a flow channel 5 into which the liquid C2 is introduced, a joining portion J4 at which the reaction tube F3 and the flow channel 5 join together, and a reaction tube F4 connected to the downstream end part of the joining portion J4 are portions related to the second stage reaction. In the second stage reaction, first, a metallated electrophilic monosubstituted aromatic compound is produced in a joined liquid M3 which is produced by the joining of the joined liquid M2 and the liquid B2 at the joining portion J3 while the joined liquid M3 flows in the reaction tube F3, and then, an electrophilic disubstituted aromatic compound is produced in a joined liquid M4 which is produced by the joining of the joined liquid M3 and the liquid C2 at the joining portion J4 while the joined liquid M4 flows in the reaction tube F4.

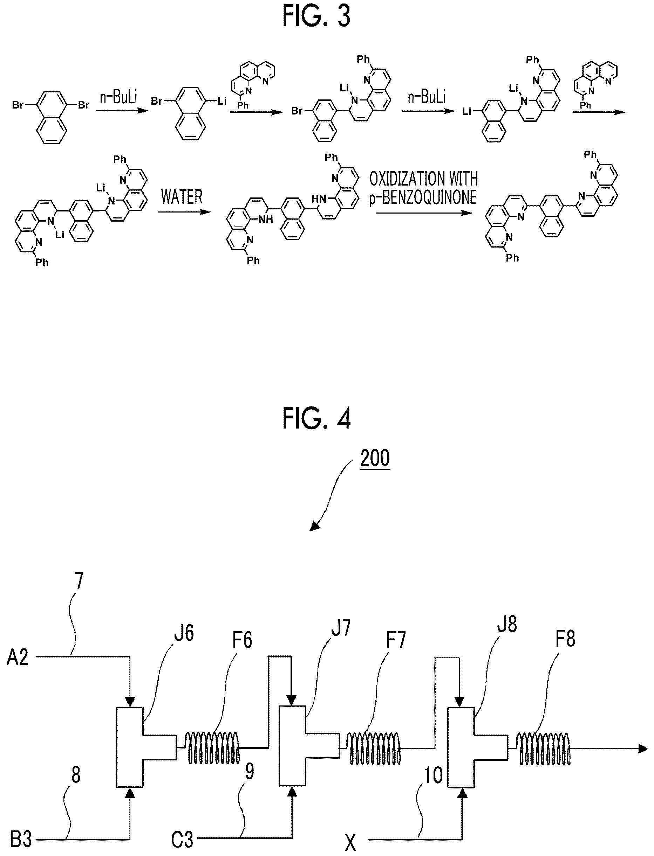

As shown in FIG. 1, the joined liquid M4 flowing in F4 is joined at a joining portion J5 with a quenching agent (X in FIG. 1) such as water or alcohol as necessary, whereby the joined liquid M4 is quenched (the metal atom in the product is converted into a hydrogen atom) while flowing in a reaction tube F5. The metallation/electrophilic substitution reaction of a halogenated aromatic compound shown in FIG. 2 is shown in more detail as shown in FIG. 3. The metal (lithium) atom in the metallated (lithiated) aromatic compound is substituted with an electrophilic compound (2-phenyl-1,10-phenanthroline), and the substituted metal (lithium) atom is present in a state of being bonded to the substitution electrophilic compound (2-phenyl-1,10-phenanthroline). That is, the compound produced in the joined liquid M4 flowing in the reaction tube F4 is a dimetallated (dilithiated) electrophilic disubstituted aromatic compound having two metal (lithium) atoms derived from an organometallic (lithium) compound, which is still highly reactive. The quenching treatment is a treatment in which the metal (lithium) atom in the dimetallated (dilithiated) electrophilic disubstituted aromatic compound is converted into a hydrogen atom, thereby terminating the reaction.

Furthermore, as necessary, the organic phase may be oxidized with an oxidizing agent such as p-benzoquinone to obtain the desired electrophilic disubstituted aromatic compound. In the metallation/electrophilic substitution reaction of a halogenated aromatic compound shown in FIGS. 2 and 3, after the quenching treatment, some of the ring-constituting nitrogen atoms of phenanthroline are present in a -NH- state, and -NH- can be returned to a -N= state (phenanthroline skeleton) by the oxidization treatment.

In the reaction schemes of FIGS. 2 and 3, a phenanthroline compound having a basic group (a ring-constituting nitrogen atom) is used as the electrophilic compound, and therefore a metal element (lithium) is in a state of being bonded to the phenanthroline skeleton. However, in a case where a different compound is used as the electrophilic compound, it is not necessarily in a state in which the metal element is bonded to the electrophilic compound. For example, in a case where chlorotrimethylsilane is used as the electrophilic compound, lithium is present mainly as lithium chloride.

In the form shown in FIG. 1, in a case where a flow-type reaction is carried out in which the reaction liquid is allowed to flow up to the reaction tube F4, this corresponds to the implementation of the manufacturing method according to the embodiment of the present invention. That is, the manufacturing method according to the embodiment of the present invention encompasses various forms, such as a form in which the flow-type reaction is carried out up to the reaction tube F4, a form in which the flow-type reaction is carried out up to the reaction tube F5, a form in which a quenching treatment is carried out in a batch manner or the like instead of the reaction tube F5, and a form in which the quenching treatment is further followed by a chemical treatment such as the above-mentioned oxidization treatment, various purification treatments, or the like depending on the purpose.

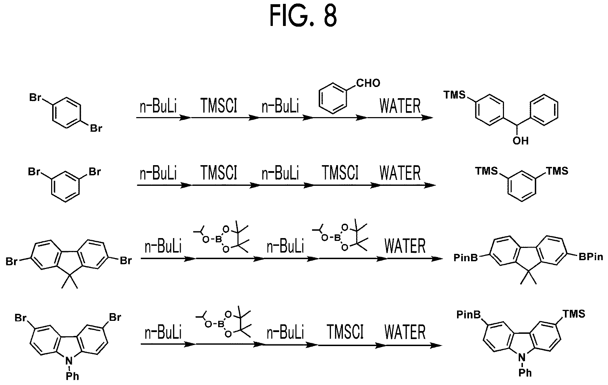

FIG. 8 shows another example of the metallation/electrophilic substitution reaction of a halogenated aromatic compound, which can be applied to a two-stage reaction system. In FIG. 8, Bu represents butyl, Ph represents phenyl, Bpin represents a pinacolatoboryl group, and TMS represents trimethylsilyl.

A flow-type reaction system 200 shown in FIG. 4 is a basic form of a one-stage reaction system in which a liquid B3 containing an organometallic compound in an organic solvent and a liquid C3 containing an electrophilic compound in an organic solvent are each introduced once into a liquid A2 containing a halogenated aromatic compound (for example, a monohalogenated aromatic compound) in an organic solvent. In the flow-type reaction system 200, at least a flow channel 7 into which the liquid A2 is introduced, a flow channel 8 into which the liquid B3 is introduced, a joining portion J6 at which the flow channel 7 and the flow channel 8 join together, a reaction tube F6 connected to the downstream end part of the joining portion J6, a flow channel 9 into which the liquid C3 is introduced, a joining portion J7 at which the reaction tube F6 and the flow channel 9 join together, and a reaction tube F7 connected to the downstream end part of the joining portion J7 constitute the one-stage reaction system. In this one-stage reaction system, first, a metallated aromatic compound is produced in a joined liquid M6 which is produced by the joining of the liquid A2 and the liquid B3 at the joining portion J6 while the joined liquid M6 flows in the reaction tube F6. Next, an electrophilic monosubstituted aromatic compound is produced in a joined liquid M7 which is produced by the joining of the joined liquid M6 and the liquid C3 at the joining portion J7 while the joined liquid M7 flows in the reaction tube F7. Thereafter, a quenching treatment or the like can be carried out as necessary in the same manner as described above. FIG. 4 shows a form in which the quenching treatment is carried out in a reaction tube F8 (a form in which the process up to the quenching treatment is continuously carried out by a flow-type reaction).

In the form shown in FIG. 4, in a case where a flow-type reaction is carried out in which the reaction liquid is allowed to flow up to the reaction tube F7, this corresponds to the implementation of the manufacturing method according to the embodiment of the present invention. That is, the manufacturing method according to the embodiment of the present invention encompasses various forms, such as a form in which the flow-type reaction is carried out up to the reaction tube F7, a form in which the flow-type reaction is carried out up to the reaction tube F8, a form in which a quenching treatment is carried out in a batch manner or the like instead of the reaction tube F8, and a form in which the quenching treatment is further followed by a chemical treatment such as the above-mentioned oxidization treatment, various purification treatments, or the like depending on the purpose.

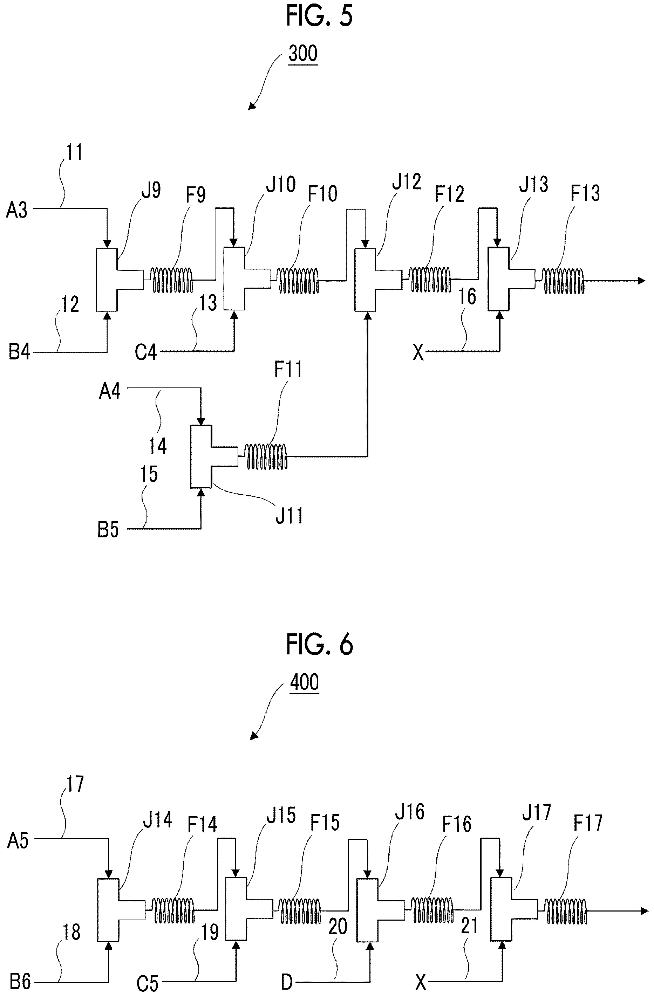

FIG. 5 shows a modification example encompassed by the manufacturing method according to the embodiment of the present invention. A flow-type reaction system 300 shown in FIG. 5 is a modification example of a one-stage reaction system. That is, the liquid containing a halogenated aromatic compound (for example, a monohalogenated aromatic compound) which is a starting material in the present invention is positioned as the liquid A4 instead of the liquid A3. The liquid A3 is positioned as a raw material for a reaction that produces an electrophilic compound for electrophilic substitution of the halogenated aromatic compound in the liquid A4. Therefore, this form is classified as a one-stage reaction system in the present invention.

In a case where the flow-type reaction is carried out using the flow-type reaction system 300 shown in FIG. 5, a liquid A3 and a liquid A4 each containing a halogenated aromatic compound in an organic solvent, a liquid B4 and a liquid B5 each containing an organometallic compound in an organic solvent, and a liquid C4 containing an electrophilic compound in an organic solvent are prepared. The flow-type reaction system 300 includes a flow channel 11 into which the liquid A3 is introduced, a flow channel 12 into which the liquid B4 is introduced, a joining portion J9 at which the flow channel 11 and the flow channel 12 join together, a reaction tube F9 connected to the downstream end part of the joining portion J9, a flow channel 13 into which the liquid C4 is introduced, a joining portion J10 at which the reaction tube F9 and the flow channel 13 join together, and a reaction tube F10 connected to the downstream end part of the joining portion J10.

The flow-type reaction system 300 further includes a flow channel 14 into which the liquid A4 is introduced, a flow channel 15 into which the liquid B5 is introduced, a joining portion J11 at which the flow channel 14 and the flow channel 15 join together, a reaction tube F11 connected to the downstream end part of the joining portion J11, a joining portion J12 at which the reaction tube F10 and the reaction tube F11 join together, and a reaction tube F12 connected to the downstream end part of the joining portion J12.

In this reaction system, first, a metallated aromatic compound is produced in a joined liquid M9 which is produced by the joining of the liquid A3 and the liquid B4 at the joining portion J9 while the joined liquid M9 flows in the reaction tube F9, and then, an electrophilic monosubstituted aromatic compound (i) is produced in a joined liquid M10 which is produced by the joining of the joined liquid M9 and the liquid C4 at the joining portion J10 while the joined liquid M10 flows in the reaction tube F10. This electrophilic monosubstituted aromatic compound (i) is not the desired compound in the present invention, but is a compound that functions as an electrophilic compound in a subsequent reaction.

In addition, separately from this, a metallated aromatic compound is produced in a joined liquid M11 which is produced by the joining of the liquid A4 containing a halogenated aromatic compound which is a starting material in the present invention and the liquid B5 at the joining portion J11 while the joined liquid M11 flows in the reaction tube F11, and then, the electrophilic monosubstituted aromatic compound (i) in the joined liquid M10 acts on the metallated aromatic compound in the joined liquid M11 to produce a desired electrophilic monosubstituted aromatic compound in a joined liquid 12 which is produced by the joining of the joined liquid M11 and the joined liquid M10 at the joining portion J12 while the joined liquid 12 flows in the reaction tube F12. Therefore, the electrophilic compound in the liquid C4 usually has, in addition to a substituent for electrophilic substitution of the metallated aromatic compound in the joined liquid M9, a substituent for electrophilic substitution of the metallated aromatic compound produced in the joined liquid 11 after the electrophilic substitution reaction. Thereafter, a quenching treatment or the like can be carried out as necessary in the same manner as described above. FIG. 5 shows a form in which the quenching treatment is carried out in a reaction tube F13 (a form in which the process up to the quenching treatment is continuously carried out by a flow-type reaction).

In the form shown in FIG. 5, in a case where a flow-type reaction is carried out in which the reaction liquid is allowed to flow up to the reaction tube F12, this corresponds to the implementation of the manufacturing method according to the embodiment of the present invention. That is, the manufacturing method according to the embodiment of the present invention encompasses various forms, such as a form in which the flow-type reaction is carried out up to the reaction tube F12, a form in which the flow-type reaction is carried out up to the reaction tube F13, a form in which a quenching treatment is carried out in a batch manner or the like instead of the reaction tube F13, and a form in which the quenching treatment is further followed by a chemical treatment such as the above-mentioned oxidization treatment, various purification treatments, or the like depending on the purpose.

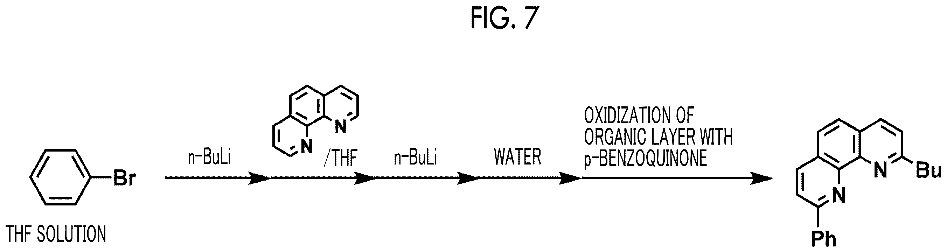

FIG. 6 shows another modification example encompassed by the manufacturing method according to the embodiment of the present invention. A flow-type reaction system 400 shown in FIG. 6 is a modification example of a one-stage reaction system. That is, an electrophilic monosubstituted aromatic compound is obtained in a one-stage reaction system using a halogenated aromatic compound (for example, a monohalogenated aromatic compound) as a starting material. This is a flow-type reaction system in which, as a reaction separate from the metallation/electrophilic substitution reaction of a halogenated aromatic compound, a nucleophilic compound (nucleophilic reagent) is then allowed to act on the resulting electrophilic monosubstituted aromatic compound to obtain an electrophilic monosubstituted/ nucleophilic substituted aromatic compound. This form is classified as a one-stage reaction system in the present invention. In addition, FIG. 7 shows an example of a reaction that is applied to the flow-type reaction system of FIG. 6. Bromoaryl (preferably bromobenzene) is used as the halogenated aromatic compound, n-butyl lithium is used as the organometallic compound, 1,10-phenanthroline is used as the electrophilic compound, and n-butyl lithium is also used as the nucleophilic compound.

In a case where the flow-type reaction is carried out using the flow-type reaction system 400 shown in FIG. 6, a liquid A5 containing a halogenated aromatic compound in an organic solvent, a liquid B6 containing an organometallic compound in an organic solvent, a liquid C5 containing an electrophilic compound in an organic solvent, and a liquid D containing a nucleophilic compound in an organic solvent are prepared. The flow-type reaction system 400 includes a flow channel 17 into which the liquid A5 is introduced, a flow channel 18 into which the liquid B6 is introduced, a joining portion J14 at which the flow channel 17 and the flow channel 18 join together, a reaction tube F14 connected to the downstream end part of the joining portion J14, a flow channel 19 into which the liquid C5 is introduced, a joining portion J15 at which the reaction tube F14 and the flow channel 19 join together, a reaction tube F15 connected to the downstream end part of the joining portion J15, a flow channel 20 into which the liquid D is introduced, a joining portion J16 at which the reaction tube F15 and the flow channel 20 join together, and a reaction tube F16 connected to the downstream end part of the joining portion J16.

In this reaction system, first, a metallated aromatic compound is produced in a joined liquid M14 which is produced by the joining of the liquid A5 and the liquid B6 at the joining portion J14 while the joined liquid M14 flows in the reaction tube F14. Next, an electrophilic monosubstituted aromatic compound is produced in a joined liquid M15 which is produced by the joining of the joined liquid M14 and the liquid C5 at the joining portion J15 while the joined liquid M15 flows in the reaction tube F15. Next, an electrophilic monosubstituted/nucleophilic substituted aromatic compound is produced in a joined liquid 16 which is produced by the joining of the joined liquid M15 and the liquid D at the joining portion J16 while the joined liquid 16 flows in the reaction tube F16. Thereafter, a quenching treatment or the like can be carried out as necessary in the same manner as described above. FIG. 6 shows a form in which the quenching treatment is carried out in a reaction tube F17 (a form in which the process up to the quenching treatment is continuously carried out by a flow-type reaction).

The moisture content of the liquid D is preferably controlled to 200 ppm or less, as in the above-mentioned liquids (A-I), (B-I), and (C-I). In addition, the more preferred range of the moisture content of the liquid D is also the same as the preferred ranges of the moisture contents of the above-mentioned liquids (A-I), (B-I), and (C-I).

In the form shown in FIG. 6, in a case where a flow-type reaction is carried out in which the reaction liquid is allowed to flow up to the reaction tube F15, this corresponds to the implementation of the manufacturing method according to the embodiment of the present invention. That is, the manufacturing method according to the embodiment of the present invention encompasses various forms, such as a form in which the flow-type reaction is carried out up to the reaction tube F15, a form in which the flow-type reaction is carried out up to the reaction tube F16, a form in which the flow-type reaction is carried out up to the reaction tube F17, a form in which a quenching treatment is carried out in a batch manner or the like instead of the reaction tube F17, and a form in which the quenching treatment is further followed by a chemical treatment such as the above-mentioned oxidization treatment, various purification treatments, or the like depending on the purpose.

In the above description, the case where the halogenated aromatic compound used in the one-stage reaction system is a monohalogenated aromatic compound has been described as an example, but the halogenated aromatic compound to be applied to the one-stage reaction system may be an aromatic compound having a plurality of halogen elements in one molecule, such as a dihalogenated aromatic compound or a trihalogenated aromatic compound. Even in a case where such halogenated aromatic compounds are used, as long as the finally obtained compound is an electrophilic monosubstituted aromatic compound, the reaction system is a one-stage reaction system.

Similarly, the halogenated aromatic compound used in the two-stage reaction system is not limited to a dihalogenated aromatic compound and may be an aromatic compound having three or more halogen elements in one molecule.

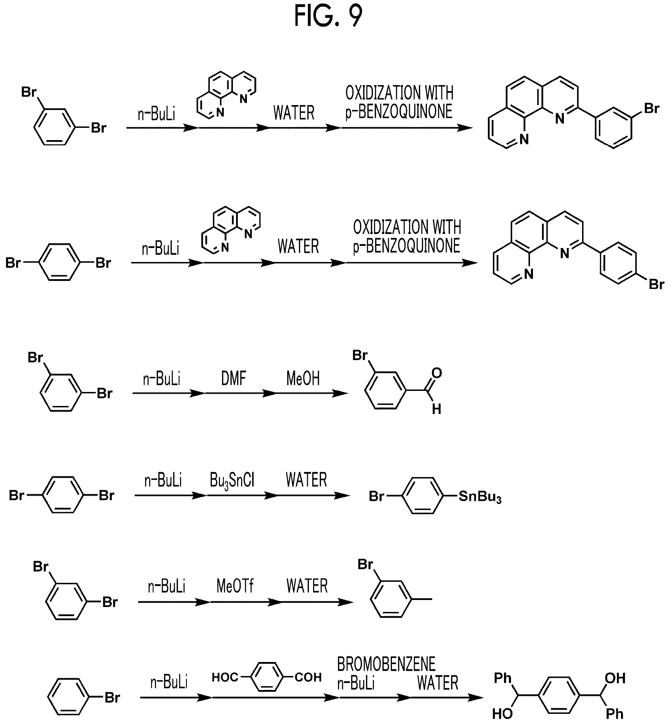

FIG. 9 shows another example of the metallation/electrophilic substitution reaction of a halogenated aromatic compound, which can be applied to a one-stage reaction system. Me represents methyl, DMF represents dimethylformamide, and MeOTf represents methyl trifluoromethanesulfonate.

In the manufacturing method according to the embodiment of the present invention, in a case where the flow-type reaction is a one-stage reaction system (in a case of a reaction in which the electrophilic substituted aromatic compound obtained from a halogenated aromatic compound is an electrophilic monosubstituted aromatic compound), it can be configured such that the electrophilic compound is used (joined) in an amount of preferably 0.80 molar equivalents or more and less than 1.20 molar equivalents, more preferably 0.80 molar equivalents or more and 1.10 molar equivalents or less, still more preferably 0.85 molar equivalents or more and 1.05 molar equivalents or less, and even still more preferably 0.90 molar equivalents or more and 1.00 molar equivalents or less with respect to the halogenated aromatic compound. Here, the phrase "using Y molar equivalents of an electrophilic compound with respect to a halogenated aromatic compound" means that, in a case where it is assumed that a liquid containing the halogenated aromatic compound in an organic solvent, which is introduced into a flow channel, is joined with a liquid containing the electrophilic compound in an organic solvent, in a state where the halogenated aromatic compound in the liquid does not react at all, to form a uniform unreacted joined liquid, the concentration of the electrophilic compound in the liquid containing the electrophilic compound in an organic solvent, the flow velocity of each liquid, and the like are controlled such that the amount of the electrophilic compound with respect to the halogenated aromatic compound in the assumed joined liquid is Y molar equivalents.

In a case where the one-stage reaction system is of the form shown in FIG. 4, a metallated aromatic compound is produced in the joined liquid M6 of the liquid A2 and the liquid B3 while the joined liquid M6 flows in the reaction tube F6. Therefore, the phrase "with respect to the halogenated aromatic compound" is roughly equivalent to the amount of the metallated aromatic compound being used as a reference. That is, in the joined liquid M7 immediately after the liquid C3 is joined, the amount of the electrophilic compound with respect to the metallated aromatic compound can be set to the above-mentioned preferred molar equivalent range.

In a case where the one-stage reaction system is of the form shown in FIG. 5, the liquid containing a halogenated aromatic compound that is a starting material in the present invention is the liquid A4 instead of the liquid A3 as described above. Since a metallated aromatic compound is produced in the joined liquid M11 of the liquid A4 and the liquid B5 while the joined liquid M11 flows in the reaction tube F11, the phrase "with respect to the halogenated aromatic compound" is roughly equivalent to the amount of the metallated aromatic compound being used as a reference. That is, the amount of the electrophilic compound (electrophilic monosubstituted aromatic compound (i)) produced in the joined liquid M10 can be set to be in the above-mentioned preferred molar equivalent range with respect to the amount of the metallated aromatic compound, immediately after the joining in the joining portion J12.

In a case where the one-stage reaction system is of the form shown in FIG. 6, a metallated aromatic compound is produced in the joined liquid M14 of the liquid A5 and the liquid B6 while the joined liquid M14 flows in the reaction tube F14. Therefore, the phrase "with respect to the halogenated aromatic compound" is roughly equivalent to the amount of the metallated aromatic compound being used as a reference. That is, in the joined liquid M15 immediately after the liquid C5 is joined, the amount of the electrophilic compound with respect to the metallated aromatic compound can be set to the above-mentioned preferred molar equivalent range.

In a case where the flow-type reaction is a multi-stage reaction system (in a case of a reaction in which one molecule of the electrophilic substituted aromatic compound obtained from a halogenated aromatic compound has two or more substituents derived from an electrophilic compound), it can be configured such that the electrophilic compound is supplied to a final stage reaction in an amount of preferably 0.80 molar equivalents or more and less than 2.00 molar equivalents, more preferably 0.80 molar equivalents or more and 1.50 molar equivalents or less, still more preferably 0.80 molar equivalents or more and 1.20 molar equivalents or less, and even still more preferably 0.90 molar equivalents or more and 1.10 molar equivalents or less with respect to the halogenated aromatic compound. Here, the phrase "an electrophilic compound in an amount of Y molar equivalents with respect to a halogenated aromatic compound is supplied to a final stage reaction" means that, in a case where it is assumed that, in a state where the halogenated aromatic compound in a liquid containing the halogenated aromatic compound in an organic solvent, which is introduced into a flow channel, does not react at all, the liquid containing the halogenated aromatic compound in an organic solvent joins together with a liquid containing the electrophilic compound in an organic solvent in the final stage to form a uniform joined liquid, the concentration of the electrophilic compound in the liquid containing the electrophilic compound which is joined in the final stage, the flow velocity of each liquid, and the like are controlled such that, in the assumed joined liquid, the amount of the electrophilic compound that has joined in the final stage is Y molar equivalents with respect to the halogenated aromatic compound.

For example, in a case where a two-stage reaction system shown in FIG. 1 is described as an example of a multi-stage reaction system, it is considered that what is subjected to the final stage reaction (the second stage reaction) is substantially the electrophilic monosubstituted halogeno-aromatic compound contained in the joined liquid M2. That is, a form in which the amount of the electrophilic compound contained in the liquid C2 (the amount of the electrophilic compound derived from the liquid C2 immediately after the joining at the joining portion J4) with respect to the metallated electrophilic monosubstituted aromatic compound produced by the reaction of the electrophilic monosubstituted halogeno-aromatic compound with the organometallic compound in the liquid B2 is set to the above-mentioned preferred range of molar equivalents is approximately a preferred form as the embodiment of the two-stage reaction system.

As described above, according to the manufacturing method of the embodiment of the present invention, it is possible to obtain the desired electrophilic monosubstituted aromatic compound at a desired high production rate even in a case where the amount of the electrophilic compound used is reduced.

In addition, in a case where the flow-type reaction is a multi-stage reaction system, in a reaction in a stage before the final stage (a reaction in each stage other than the final stage), it can be configured such that the electrophilic compound is supplied to the reaction in a stage before the final stage, in an amount of preferably 0.80 molar equivalents or more and less than 1.20 molar equivalents, more preferably 0.80 molar equivalents or more and 1.10 molar equivalents or less, still more preferably 0.85 molar equivalents or more and 1.05 molar equivalents or less, and even still more preferably 0.90 molar equivalents or more and 1.00 molar equivalents or less with respect to the halogenated aromatic compound. For example, referring to FIGS. 1 to 3, in the reaction in a stage before the final stage (the first stage reaction), the halogenated aromatic compound is a dihalogenated aromatic compound contained in the liquid A1. Therefore, it is preferable that the amount of the electrophilic compound contained in the liquid C1 with respect to the amount of the dihalogenated aromatic compound contained in the liquid A1 is within the above-mentioned preferred molar equivalent range immediately after the joining at the joining portion J2.

The amount of the organometallic compound used in the manufacturing method according to the embodiment of the present invention will be described.

In a case where the flow-type reaction is a one-stage reaction system, it can be configured such that the organometallic compound is used (joined) in an amount of preferably 0.80 molar equivalents or more and less than 1.20 molar equivalents, more preferably 0.85 molar equivalents or more and 1.15 molar equivalents or less, still more preferably 0.90 molar equivalents or more and 1.10 molar equivalents or less, even still more preferably 1.00 molar equivalents or more and 1.05 molar equivalents or less, and even still further more preferably 1.02 molar equivalents or more and 1.05 molar equivalents or less with respect to the halogenated aromatic compound.

In addition, in a case where the flow-type reaction is a multi-stage reaction system, in the reaction in each stage other than the final stage, the amount of the organometallic compound to be supplied to each stage can be set to preferably 0.80 molar equivalents or more and less than 2.00 molar equivalents, more preferably 0.90 molar equivalents or more and 1.50 molar equivalents or less, still more preferably 1.00 molar equivalents or more and 1.20 molar equivalents or less, even still more preferably 1.00 molar equivalents or more and 1.10 molar equivalents or less, and even still further more preferably 1.02 molar equivalents or more and 1.05 molar equivalents or less with respect to the halogenated aromatic compound.

In addition, in a case where the flow-type reaction is a multi-stage reaction system, in the final stage reaction, the amount of the organometallic compound to be supplied to the final stage can be set to preferably 0.80 molar equivalents or more and less than 2.00 molar equivalents, more preferably 0.90 molar equivalents or more and 1.20 molar equivalents or less, still more preferably 1.00 molar equivalents or more and 1.10 molar equivalents or less, and even still more preferably 1.02 molar equivalents or more and 1.05 molar equivalents or less with respect to the halogenated aromatic compound.

Next, the configuration of each member, reagent, and the like in the flow-type reaction will be described, but the present invention is not limited to the form described below except as specified in the present invention.

### <Flow channel>

It is preferable that the equivalent diameter of each of the flow channels (flow channels 1 to 21) through which each liquid flows is set to 0.2 to 50 mm. By setting the equivalent diameter of the flow channel to 0.2 mm or more, it is possible to suppress an increase in pressure during feeding of liquid, and it is also possible to suppress the clogging of the flow channel even in a case where insoluble matter is produced. In addition, by setting the equivalent diameter of each flow channel to 50 mm or less, the liquid temperature at the joining portion can be appropriately controlled. The equivalent diameter of each flow channel is more preferably 0.5 to 30 mm, still more preferably 1 to 20 mm, and even still more preferably 1 to 10 mm.

The "equivalent diameter" is a term used in the field of mechanical engineering. In a case where an equivalent circular tube is assumed for a pipe or flow channel having a given inner cross-sectional shape of the tube, the diameter of the inner cross section of the equivalent circular tube is called the equivalent diameter. The equivalent diameter (deq) is defined as deq = 4A/p in which A represents an inner cross-sectional area of a pipe and p represents a wetted perimeter (inner perimeter) of a pipe. In a case of being applied to a circular tube, the equivalent diameter corresponds to the diameter of the inner cross section of the circular tube. Based on the data regarding an equivalent circular tube, the equivalent diameter is used for estimating the fluidity or heat transfer characteristics of the pipe, and represents the spatial scale (representative length) of a phenomenon. The equivalent diameter is deq = 4a²/4a = a for a square tube in which a represents one side of the inner cross section of the tube; deq = a/3^{1/2} for an equilateral triangular tube in which a represents one side thereof; and deq = 2h for a flow between parallel flat plates in which h represents a height of a flow channel (see, for example, "Mechanical Engineering Dictionary", edited by The Japan Society of Mechanical Engineers, 1997, Maruzen Co., Ltd).

The length of each flow channel is not particularly limited, and for example, the flow channel can be configured by a tube having a length of about 10 cm to 15 m (preferably 30 cm to 10 m).

The material of the tube is not particularly limited, and examples thereof include perfluoroalkoxyalkane (PFA), Teflon (registered trademark), an aromatic polyether ketone-based resin, stainless steel, copper or a copper alloy, nickel or a nickel alloy, titanium or a titanium alloy, quartz glass, and lime soda glass. From the viewpoint of flexibility and chemical resistance, the material of the tube is preferably PFA, Teflon (registered trademark), stainless steel, a nickel alloy, or titanium.

The flow velocity of each liquid introduced into each flow channel is not particularly limited, and can be appropriately set depending on the purpose in consideration of the equivalent diameter of the flow channel, the reaction amount ratio, the liquid concentration, and the like. The flow velocity of each liquid is, for example, preferably 0.1 to 5000 mL/min, more preferably 0.5 to 3000 mL/min, still more preferably 1 to 3000 mL/min, and even still more preferably 100 to 2000 mL/min.

### (Liquid containing halogenated aromatic compound in organic solvent)

In the liquid (A-I) containing a halogenated aromatic compound in an organic solvent, the type of the organic solvent to be used is not particularly limited as long as it has an ability to dissolve the halogenated aromatic compound. Examples of the organic solvent include a halogen-containing solvent, an ether solvent having a linear, branched, or cyclic structure, and a hydrocarbon solvent.

Examples of the halogen-containing solvent include methylene chloride, chloroform, dichloroethane, carbon tetrachloride, chlorobenzene, and o-dichlorobenzene.

Examples of the ether solvent include tetrahydrofuran (THF), dioxane, methyl tertiary butyl ether, cyclopentyl methyl ether, ethylene glycol dibutyl ether, diethylene glycol dimethyl ether, diethylene glycol diethyl ether, diethylene glycol dibutyl ether, and derivatives thereof.

Examples of the hydrocarbon solvent include hexane, heptane, octane, cyclohexane, methylcyclohexane, benzene, toluene, xylene, mesitylene, decalin, tetralin, and derivatives thereof.

In addition, a ketone-based solvent such as acetone, methyl ethyl ketone, diisobutyl ketone, cyclohexanone, or methyl isobutyl ketone, a nitrile-based solvent such as acetonitrile, a lactone-based solvent such as γ-butyrolactone, an ester-based solvent such as ethyl acetate or butyl acetate, and an amide-based solvent such as dimethylacetamide, dimethylformamide, N-methyl-2-pyrrolidone, or 1,3-dimethyl-2-imidazolidinone can also be used as the organic solvent.

The above-mentioned organic solvents may be used alone or in a mixture of two or more thereof.

Above all, it is preferable to use at least one of methylene chloride, chloroform, chlorobenzene, o-dichlorobenzene, tetrahydrofuran, dioxane, toluene, xylene, mesitylene, cyclohexanone, methyl ethyl ketone, or acetonitrile, and it is more preferable to use at least one of methylene chloride, chlorobenzene, o-dichlorobenzene, tetrahydrofuran, toluene, xylene, mesitylene, or acetonitrile.

In the liquid (A-I), the type of the halogenated aromatic compound is not particularly limited and is appropriately selected depending on the chemical structure of the desired compound and the like. The number, type, position, and the like of halogen elements in the halogenated aromatic compound are not particularly limited. Examples of the halogen element include fluorine, chlorine, bromine, and iodine, among which bromine is preferable. Examples of the ring structure of the halogenated aromatic compound include monocyclic or polycyclic 6- to 10-membered aromatic hydrocarbon rings, such as benzene, naphthalene, anthracene, phenanthrene, and fluorene; and monocyclic or polycyclic 5- to 10-membered aromatic heterocyclic rings containing 1 to 4 atoms selected from nitrogen, oxygen, and sulfur, such as pyrrole, furan, thiophene, imidazole, pyrazole, oxazole, thiazole, carbazole, pyridine, pyrazine, pyrimidine, pyridazine, and triazine.

The concentration of the halogenated aromatic compound in the liquid (A-I) is appropriately set in consideration of the concentration of the liquid (B-I), (C-I), or the like, the flow velocity of each liquid, and the like. The concentration of the halogenated aromatic compound in the liquid (A-I) can be set to, for example, 0.1 to 10 mol/L (hereinafter, also referred to as M), and is preferably set to 0.3 to 8 mol/L.

### (Liquid containing organometallic compound in organic solvent)

In the liquid (B-I) containing an organometallic compound in an organic solvent, the type of solvent to be used is not particularly limited as long as it has an ability to dissolve the organometallic compound. For example, the organic solvents described above as the organic solvent that can be used for the liquid (A-I) can also be suitably used as the organic solvent for the liquid (B-1).

In the liquid (B-I), the type of the organometallic compound is not particularly limited and is not particularly limited as long as it can metallate a halogenated aromatic compound. For example, an organolithium compound and an organomagnesium compound can be used, with an organolithium compound being preferred. The organolithium compound is not particularly limited, and can be appropriately selected from conventionally known organolithium compounds. Examples of the organolithium compound include alkyl lithium such as methyl lithium, ethyl lithium, propyl lithium, butyl lithium (n-butyl lithium, sec-butyl lithium, iso-butyl lithium, tert-butyl lithium, or the like), pentyl lithium, hexyl lithium, methoxymethyl lithium, or ethoxymethyl lithium; benzyl lithium such as α-methylstyryl lithium, 1,1-diphenyl-3-methylpentyl lithium, or 3-methyl-1,1-diphenylpentyl lithium; alkenyl lithium such as vinyl lithium, allyl lithium, propenyl lithium, or butenyl lithium; alkynyl lithium such as ethynyl lithium, butynyl lithium, pentynyl lithium, or hexynyl lithium; aralkyl lithium such as benzyl lithium or phenyl ethyl lithium; aryl lithium such as phenyl lithium or naphthyl lithium; heterocyclic lithium such as 2-thienyl lithium, 4-pyridyl lithium, or 2-quinolyl lithium; and an alkyl lithium-magnesium complex such as tri(n-butyl)magnesium lithium or trimethyl magnesium lithium. Above all, from the viewpoint of high reactivity and the ability to speed up an initiation reaction, alkyl lithium is more preferable, and n-butyl lithium is particularly preferable.

The concentration of the organometallic compound in the liquid (B-I) is appropriately set in consideration of the concentration of the liquid (A-I), (C-I), or the like, the flow velocity of each liquid, and the like. The concentration of the organometallic compound in the liquid (B-I) can be set to, for example, 0.5 to 5 mol/L, and is preferably set to 1 to 3 mol/L.

### (Liquid containing electrophilic compound in organic solvent)

In the liquid (C-I) containing an electrophilic compound in an organic solvent, the type of the electrophilic compound is not particularly limited as long as it is a compound having a functional group with an electron-accepting ability (that is, a compound which can act as an electrophile). The electrophilic compound that can be used in the metallation reaction by a halogen-metal exchange reaction using an organometallic compound is appropriately selected depending on the chemical structure of the desired compound and the like. Examples of the electrophilic compound include
halogens such as chlorine, bromine, and iodine;
inorganic substances such as sulfur, sulfur dioxide, and oxygen;
carbon dioxide;
sulfonic acids such as trifluoromethylsulfonic acid methyl ester and trifluoromethylbenzenesulfonic acid;
dimethyl sulfate;
nitriles such as acetonitrile, propionitrile, and benzonitrile;
imines such as benzophenone imine and acetophenone imine;
silicon halides such as chlorotrimethylsilane, chlorodimethylphenylsilane, chlorodimethylsilane, and bromotrimethylsilane;
a chlorosilane compound such as chlorodialkylhydrosilane;
boron halides such as trichloroborane and tribromoborane;
boronic acid esters such as pinacol boronic acid ester, trimethyl boronic acid ester, and triisopropyl boronic acid ester;
boron compounds such as methoxydiethylborane, tris(dimethylamino)borane, and bis(pinacolato)diborane;
tin compounds such as dibutyltin dichloride and diphenyltin dibromide;
aldehydes such as paraformaldehyde, acetaldehyde, propionaldehyde, butylaldehyde, acrylic aldehyde, benzaldehyde, and nicotinic aldehyde;
ketones such as acetone, 2-butanone, benzophenone, acetophenone, DMF, and tert-butyl-4-oxo-1-piperidinecarboxylate;
esters such as ethyl chloroformate, phenyl chloroformate, methyl formate, ethyl formate, ethyl acetate, butyl acetate, octyl acetate, phenyl acetate, methyl benzoate, ethyl benzoate, and phenyl benzoate;
acid anhydrides such as acetic acid anhydride, phthalic acid anhydride, succinic acid anhydride, and maleic acid anhydride;
acyl halides such as acetyl chloride, benzoyl chloride, and 2-pyridinecarbonyl chloride;
oxiranes such as oxirane and 2-methyl-oxirane;
aziridines such as 6-azabicyclo[3,1,0]hexane and 7-azabicyclo[4,1,0]heptane; α,β-unsaturated ketones such as 3-oxo-1,3-diphenyl-1-propene and 2-methyl-3-oxo-3-diphenyl-1-propene;
alkyl halides such as methyl iodide, ethyl iodide, butyl iodide, methyl bromide, ethyl bromide, hexyl bromide, octyl bromide, 1,2-diiodoethane, 1,2-dibromoethane, 1,6-diiodohexane, 1,8-dibromooctane, and 1,2-dibromocyclopentene;
acid imides such as N-bromosuccinimide, N-iodosuccinimide, N-chlorosuccinimide, and N-bromophthalimide;
disulfides such as dimethyldisulfide and diphenyldisulfide;
phosphines such as chlorodiphenylphosphine and chlorodimethylphosphine;
phosphine oxides such as chlorodiphenylphosphine oxide and chlorodimethylphosphine oxide and
heterocyclic compounds such as pyridine, pyrimidine, pyridazine, phenanthroline, bipyridine, terpyridine, imidazole, pyrazole, oxazole, and thiazole.

The concentration of the halogenated aromatic compound in the liquid (C-I) is appropriately set in consideration of the concentration of the liquid (A-I), (B-I), or the like, the flow velocity of each liquid, and the like. The concentration of the halogenated aromatic compound in the liquid (C-I) can be set to, for example, 0.1 to 10 mol/L, and is preferably set to 0.3 to 3 mol/L.

The flow velocity at which each of the above-mentioned liquids flows in the flow channel is not particularly limited, and can be appropriately set depending on the purpose in consideration of the equivalent diameter of the flow channel, the concentration of the solution, and the like. The flow velocity is, for example, preferably 0.1 to 5000 mL/min, more preferably 0.5 to 3000 mL/min, still more preferably 1 to 3000 mL/min, and even still more preferably 100 to 2000 mL/min.

### <Joining portion>

The material of each of the joining portions (joining portions J1 to J17) is not particularly limited. For example, it is possible to use the joining portion consisting of materials such as perfluoroalkoxyalkane (PFA), Teflon (registered trademark), an aromatic polyether ketone-based resin, stainless steel, copper or a copper alloy, nickel or a nickel alloy, titanium or a titanium alloy, quartz glass, and lime soda glass.

As each joining portion, for example, those commercially available as a T-shaped connector, a Y-shaped connector, or a multilayer tubular connector can be widely used. In addition, a connector having a cross-shaped or more branches can be appropriately used as necessary.

### <Reaction tube>

The shape of the reaction tube (reaction tubes F1 to F17) is not particularly limited, and a tube is usually used. The preferred material for the reaction tube is the same as the preferred material for the flow channel described above. In addition, the reaction time can be adjusted by the equivalent diameter and length of the reaction tube, the flow rate setting of the liquid feeding pump, and the like. Usually, the equivalent diameter of the reaction tube is preferably 0.1 to 50 mm, more preferably 0.2 to 20 mm, still more preferably 0.4 to 15 mm, even still more preferably 0.7 to 12 mm, and even still further more preferably 1 to 10 mm. In addition, the length of the reaction tube is preferably 0.5 to 50 m and more preferably 1 to 30 m.

With regard to the temperature of the reaction tube (that is, the reaction temperature), the temperature of the reaction tube at which metallation occurs through the reaction with the organometallic compound is preferably set to -80°C to 100°C and more preferably set to -30°C to 50°C. In addition, the temperature of the reaction tube at which the substitution reaction of the metallation site by the electrophilic compound occurs is preferably set to -50°C to 120°C and more preferably set to -20°C to 80°C.

The temperature of the joining portion or flow channel in the front section of the reaction tube is appropriately adjusted so that the temperature of the reaction tube can be precisely controlled. Usually, it is preferable that the temperature of the flow channel or the joining part and the temperature of the reaction tube connected to the downstream thereof are set to the same temperature.

The retention time (reaction time) of each joined liquid in the reaction tube is preferably set to 0.1 seconds or more, more preferably set to 1 to 600 seconds, and still more preferably set to 3 to 200 seconds.

Hereinafter, the present invention will be described in more detail with reference to Examples, but the present invention is not limited to those Examples.

### Examples

### [Reference Example 1]

According to the reaction schemes shown in FIG. 2 and FIG. 3, a desired electrophilic substituted aromatic compound (desired compound) was prepared using the flow-type reaction system 100 having the configuration shown in FIG. 1, in which a part of the T-shaped connector was changed to a cross connector as described below. In Reference Example 1, as the solvent for each solution described below that was allowed to flow in the flow channel, a mixture of industrial THF itself and THF from which moisture had been removed to reduce the moisture content to prepare the moisture content, or a mixture of THF in which the moisture content had been prepared by carrying out a dehydration treatment was appropriately used. In addition, the moisture content shown in the tables which will be given later indicates the moisture content of the solution of the halogenated aromatic compound and the solution of the electrophilic compound. The solution of the organometallic compound is anhydrous. This also applies to each of Reference Examples, Comparative Examples, and Examples which will be described later.

Specific reaction conditions are as follows.

### Liquid feeding pump (not shown in drawing):

Five syringe pumps PHD ULTRA manufactured by Harvard Apparatus and one dual pump KP-22 manufactured by FLOM Corporation were used.

### Temperature control:

The temperatures of the flow channels 1 and 2, the joining portion J1, and the reaction tube F1 were set to 0°C.

The temperatures of the flow channel 3, the joining portion J2, and the reaction tube F2 were set to 0°C.

The temperatures of the flow channel 4, the joining portion J3, and the reaction tube F3 were set to -20°C.

The temperatures of the flow channel 5, the joining portion J4, and the reaction tube F4 were set to 5°C.

The temperatures of the flow channel 6, the joining portion J5, and the reaction tube F5 were set to 5°C.

### Flow channel 1:

The flow channel 1 had a structure established by dividing a single SUS tube into two parts by using a T-shaped connector. More specifically, a T-shaped connector (inner diameter: 1.0 mm) was connected to an SUS316 tube having an outer diameter of 1/16 inches, an inner diameter of 1.0 mm, and a length of 50 cm, and two SUS316 tubes having an outer diameter of 1/16 inches, an inner diameter of 1.0 mm, and a length of 3 cm were connected to the T-shaped connector to face each other, thus forming a flow channel 1.

Flow channel 2, flow channel 3, flow channel 4, flow channel 5, and flow channel 6:
An SUS316 tube having an outer diameter of 1/16 inches, an inner diameter of 1.0 mm, and a length of 50 cm was used.

### Reaction tube F1:

An SUS316 tube having an outer diameter of 1/16 inches, an inner diameter of 1.0 mm, and a length of 50 cm was used.

### Reaction tube F2:

The reaction tube F2 had a structure established by dividing a single SUS tube into two parts by using a T-shaped connector. More specifically, a T-shaped connector (inner diameter: 1.0 mm) was connected to an SUS316 tube having an outer diameter of 1/16 inches, an inner diameter of 1.0 mm, and a length of 2.0 m, and two SUS316 tubes having an outer diameter of 1/16 inches, an inner diameter of 1.0 mm, and a length of 3 cm were connected to the T-shaped connector to face each other, thus forming a reaction tube F2.

### Reaction tube F3:

An SUS316 tube having an outer diameter of 1/16 inches, an inner diameter of 1.0 mm, and a length of 2.0 m was used.

### Reaction tube F4:

An SUS316 tube having an outer diameter of 1/16 inches, an inner diameter of 1.0 mm, and a length of 1.5 m was used.

### Reaction tube F5:

A PTFE tube having an outer diameter of 1/8 inches, an inner diameter of 1.6 mm, and a length of 1.5 m was used.

### Joining portion J1:

A cross connector having an inner diameter of 0.5 mm was used. Two SUS316 tubes connected to the T-shaped connector constituting the flow channel 1 to face each other were respectively connected to two connection ports facing each other among four connection ports of the cross connector. The flow channel 2 was connected to one of the remaining two connection ports and the remaining connection port was used as a discharge port for discharging the liquid.

### Joining portion J2:

A T-shaped connector having an inner diameter of 0.5 mm was used. The reaction tube F1 and the flow channel 3 were respectively connected to two connection ports facing each other among three connection ports of the T-shaped connector. The remaining connection port was used as a discharge port for discharging the liquid.

### Joining portion J3:

A cross connector having an inner diameter of 0.5 mm was used. Two SUS316 tubes connected to the T-shaped connector constituting the reaction tube F2 to face each other were respectively connected to two connection ports facing each other among four connection ports of the cross connector. The flow channel 4 was connected to one of the remaining two connection ports and the remaining connection port was used as a discharge port for discharging the liquid.

### Joining portion J4:

A T-shaped connector having an inner diameter of 0.5 mm was used. The reaction tube F3 and the flow channel 5 were respectively connected to two connection ports facing each other among three connection ports of the T-shaped connector. The remaining connection port was used as a discharge port for discharging the liquid.

### Joining portion J5:

A T-shaped connector having an inner diameter of 1.3 mm was used. The reaction tube F4 and the flow channel 6 were respectively connected to two connection ports facing each other among three connection ports of the T-shaped connector. The remaining connection port was used as a discharge port for discharging the liquid.

### Liquid A1 containing dihalogenated aromatic compound in organic solvent:

A solution obtained by dissolving 1,4-dibromonaphthalene in THF (1,4-dibromonaphthalene solution A1, 1,4-dibromonaphthalene concentration: 0.35 M) was prepared.

### Liquid B1 containing organometallic compound in organic solvent:

n-BuLi (n-butyl lithium solution B1, n-BuLi concentration: 1.6 M, n-hexane solution) manufactured by Kanto Chemical Co., Inc. was used.

### Liquid B2 containing organometallic compound in organic solvent:

n-BuLi (n-butyl lithium solution B2, n-BuLi concentration: 1.6 M, n-hexane solution) manufactured by Kanto Chemical Co., Inc. was used.

### Liquid C1 containing electrophilic compound in organic solvent:

A solution obtained by dissolving 2-phenyl-1,10-phenanthroline in THF (2-phenyl-1,10-phenanthroline solution C1, 2-phenyl-1,10-phenanthroline concentration: 0.31 M) was prepared and used in the first stage reaction.

### Liquid C2 containing electrophilic compound in organic solvent:

A solution obtained by dissolving 2-phenyl-1,10-phenanthroline in THF (2-phenyl-1,10-phenanthroline solution C2, 2-phenyl-1,10-phenanthroline concentration: 0.31 M) was prepared and used in the second stage reaction.

### Liquid feeding conditions:

1,4-Dibromonaphthalene solution A1: 5.4 mL/min
n-Butyl lithium solution B1: 1.2 mL/min
n-Butyl lithium solution B2: 1.2 mL/min
2-Phenyl-1,10-phenanthroline solution C1: 6.0 mL/min
2-Phenyl-1,10-phenanthroline solution C2: 12.0 mL/min
Water (X): 1.7 mL/min

The analyses in each of Reference Examples, Comparative Examples, and Examples were carried out as follows.

### Measurement of moisture content of each solution:

The measurement was carried out using a trace moisture meter CA-200 manufactured by Nittoseiko Analytech Co., Ltd.

### Production rate (%) of desired compound:

The ratio of the molar amount of the desired compound to the molar amount of the introduced halogenated aromatic compound as a raw material was defined as the production rate. The production rate was determined based on the results of analysis using liquid chromatography (LC), gas chromatography (GC), or 1H-NMR under the following conditions. The production rate (%) was calculated by a calibration curve method or an internal standard method. In the Reference Examples, Comparative Examples, and Examples in which the raw material and the desired compound were the same, the production rate of the desired compound was determined by analysis under the same conditions.

### - LC conditions -

Measuring instrument: LC-2060C 3D (manufactured by Shimadzu Corporation)
Column: Mightysil RP-8 (4.6 mmφ × 250 mm, 5 µm)
Column temperature: 45°C
Eluent A: water (0.1% phosphoric acid aqueous solution)
Eluent B: acetonitrile/THF = 8/2 (volume ratio)
Flow rate: 1.0 mL/min
Detection wavelength: 254 nm
Injection volume: 5 µL

### - GC conditions -

Measuring instrument: Nexis GC-2030 (manufactured by Shimadzu Corporation)
Column: DB-17MS (30 m × 0.25 mm × 0.25 µm, manufactured by GL Sciences Inc.)
Detector temperature: 300°C
Vaporization chamber temperature: 250°C
Column flow rate: 1.0 mL/min
Carrier gas: helium
Injection volume: 1 µL

### - NMR conditions -

Measuring instrument: Ascend NMR 400 (manufactured by Bruker Corporation)
Internal standard: 1.3.5-trimethoxybenzene
Identification of the desired compound was carried out by 1H-NMR.
Chemical shifts σ (ppm) by ¹H-NMR (400 MHz, solvent: CDCl₃, internal reference substance: tetramethylsilane (TMS)) = 8.83-8.80 (2H, m), 8.44-8.41 (6H, m), 8.37-8.35 (2H, m), 8.19-8.16 (4H, m), 8.11-8.09 (2H, m), 7.91-7.87 (4H, m), 7.64-7.61 (2H, m), 7.54-7.50 (4H, m), 7.46-7.43 (2H, m)

The results are shown in the tables which will be given later. In the tables which will be given later, the molar equivalent is a molar amount with respect to 1 mole of a halogenated aromatic compound (dihalogenated aromatic compound (1,4-dibromonaphthalene)) as a raw material. In addition, the moisture content was set to 273 ppm in all of the 1,4-dibromonaphthalene solution A1 and the 2-phenyl-1,10-phenanthroline solutions C1 and C2.

**[Table 1]**

| | Moisture content (ppm) | First stage reaction | | Second stage reaction | | LC production rate (%) |
|---|---|---|---|---|---|---|
| | | Organometallic compound (molar equivalent) | Electrophilic compound (molar equivalent) | Organometallic compound (molar equivalent) | Electrophilic compound (molar equivalent) | |
| Reference Example 1 | 273 | 1.0 | 1.0 | 1.0 | 2.0 | 63 |

It was confirmed that, in the final stage (second stage) reaction, in a case where an excess amount of the electrophilic compound was used, the production rate of the desired compound was increased even in a case where the moisture content of each solution flowing in the flow channel was higher (273 ppm) than that specified in the present invention.

### [Comparative Examples 1 to 4]

The desired electrophilic substituted aromatic compound was prepared in the same manner as in Reference Example 1, except that, in Reference Example 1, the amount of the electrophilic compound used in the final stage (second stage) reaction was reduced as shown in the table below by adjusting the flow velocity of the 2-phenyl-1,10-phenanthroline solution C2.

The results are shown in the table below.

**[Table 2]**

| | Moisture content (ppm) | First stage reaction | | Second stage reaction | | LC production rate (%) |
|---|---|---|---|---|---|---|
| | | Organometallic compound (molar equivalent) | Electrophilic compound (molar equivalent) | Organometallic compound (molar equivalent) | Electrophilic compound (molar equivalent) | |
| Comparative Example 1 | 273 | 1.0 | 1.0 | 1.0 | 1.5 | 56 |
| Comparative Example 2 | 273 | 1.0 | 1.0 | 1.0 | 1.2 | 53 |
| Comparative Example 3 | 273 | 1.0 | 1.0 | 1.0 | 1.0 | 48 |
| Comparative Example 4 | 273 | 1.0 | 1.0 | 1.0 | 0.8 | 37 |

It can be seen that, in the final stage (second stage) reaction, in a case where the amount of the electrophilic compound used is smaller than that in Reference Example 1, and then in a case where the moisture content of each solution flowing in the flow channel is higher (273 ppm) than that specified in the present invention, the production rate of the desired compound decreases depending on the amount of the electrophilic compound used.

### [Examples 1 to 10]

A desired electrophilic substituted aromatic compound was prepared in the same manner as in Reference Example 1, except that the moisture content of each solution to be allowed to flow in the flow channel (the n-butyl lithium solutions B1 and B2 are anhydrous as described above, so "each solution" refers to a solution other than the n-butyl lithium solutions B1 and B2; the same applies hereinafter) was set as shown in the table which will be given later, and the amount of the electrophilic compound used in the final stage (second stage) reaction was adjusted by adjusting the flow velocity of the 2-phenyl-1,10-phenanthroline solution C2.

### [Example 11]

A desired electrophilic substituted aromatic compound was prepared in the same manner as in Example 5, except that the moisture content of each solution to be allowed to flow in the flow channel was set as shown in the table which will be given later, and the temperatures of the flow channels 4 and 5, the joining portions J3 and J4, and the reaction tubes F3 and F4 were set to 0°C.

### [Example 12]

A desired electrophilic substituted aromatic compound was prepared in the same manner as in Example 11, except that the moisture content of each solution to be allowed to flow in the flow channel was set as shown in the table which will be given later and the temperatures of the flow channel 3, the joining portion J2, and the reaction tube F2 were set to 50°C.

### [Example 13]

A desired electrophilic substituted aromatic compound was prepared in the same manner as in Example 11, except that the moisture content of each solution to be allowed to flow in the flow channel was set as shown in the table which will be given later and the temperatures of the flow channels 1 and 2, the joining portion J1, and the reaction tube F1 were set to -20°C.

### [Example 14]

A desired electrophilic substituted aromatic compound was prepared in the same manner as in Example 11, except that the moisture content of each solution to be allowed to flow in the flow channel was set as shown in the table which will be given later and the temperatures of the flow channel 5, the joining portion J4, and the reaction tube F4 were set to 50°C.

### [Example 15]

A desired electrophilic substituted aromatic compound was prepared in the same manner as in Example 5, except that the moisture content of each solution to be allowed to flow in the flow channel was set as shown in the table which will be given later and a quenching treatment was carried out in a batch manner.

### [Example 16]

A desired electrophilic substituted aromatic compound was prepared in the same manner as in Example 5, except that the moisture content of each solution to be allowed to flow in the flow channel was set as shown in the table which will be given later, the flow channel 1 was an SUS316 tube having an outer diameter of 1/16 inches, an inner diameter of 1.0 mm, and a length of 50 cm, the reaction tube F2 was an SUS316 tube having an outer diameter of 1/16 inches, an inner diameter of 1.0 mm, and a length of 2.0 m, and the joining portions J1 and J3 were T-shaped connectors (inner diameter: 0.5 mm).

### [Example 17]

A desired electrophilic substituted aromatic compound was prepared in the same manner as in Example 5, except that the moisture content of each solution to be allowed to flow in the flow channel was set as shown in the table which will be given later, and the amount of the organometallic compound used in the final stage (second stage) reaction was adjusted by adjusting the flow velocity of the n-butyl lithium solution B2.

### [Example 18]

A desired electrophilic substituted aromatic compound was prepared in the same manner as in Example 5, except that the moisture content of each solution to be allowed to flow in the flow channel was set as shown in the table which will be given later and the amount of the organometallic compound used in the first stage reaction was adjusted by adjusting the flow velocity of the n-butyl lithium solution B1.

### [Example 19]

A desired electrophilic substituted aromatic compound was prepared in the same manner as in Example 5, except that each solution to be allowed to flow in the flow channel was passed through a dehydration column disposed in the flow channel to reduce the moisture content, the temperatures of the flow channel 3, the joining portion J2, and the reaction tube F2 were set to 50°C, the temperatures of the flow channel 4, the joining portion J3, and the reaction tube F3 were set to -15°C, the temperatures of the flow channel 5, the joining portion J4, and the reaction tube F4 were set to 15°C, the joining portion J5 was a T-shaped connector having an inner diameter of 2.3 mm, and the temperatures of the flow channel 6, the joining portion J5, and the reaction tube F5 were set to 15°C.

### [Example 20]

A desired electrophilic substituted aromatic compound was prepared in the same manner as in Example 19, except that the flow channel 1 was an SUS316 tube having an outer diameter of 1/16 inches, an inner diameter of 1.0 mm, and a length of 50 cm, the reaction tube F2 was an SUS316 tube having an outer diameter of 1/16 inches, an inner diameter of 1.0 mm, and a length of 2.0 m, the joining portion J1 was a T-shaped connector having an inner diameter of 1.0 mm, the temperatures of the flow channels 1 and 2, the joining portion J1, and the reaction tube F1 were set to -15°C, and the joining portions J2, J3, and J4 were T-shaped connectors having an inner diameter of 1.0 mm.

### [Example 21]

A desired electrophilic substituted aromatic compound was prepared in the same manner as in Example 20, except that MeOH was fed as a quenching agent X at a rate of 3.4 mL/min, and the temperatures of the flow channel 6, the joining portion J5, and the reaction tube F5 were set to 0°C.

### [Example 22]

A desired electrophilic substituted aromatic compound was prepared in the same manner as in Example 20, except that the amounts of the organometallic compound and the electrophilic compound used in the first stage and second stage reactions were adjusted by adjusting the flow velocities of the n-butyl lithium solution B1, the 2-phenyl-1,10-phenanthroline solution C1, the n-butyl lithium solution B2, and the 2-phenyl-1,10-phenanthroline solution C2.

### [Example 23]

A desired electrophilic substituted aromatic compound was prepared in the same manner as in Example 22, except that the flow velocity of each liquid was adjusted to be three times that in Example 22.

### [Example 24]

A desired electrophilic substituted aromatic compound was prepared in the same manner as in Example 23, except that the flow channel 1 was an SUS316 tube having an outer diameter of 1/16 inches, an inner diameter of 1.0 mm, and a length of 50 cm, the reaction tube F2 was an SUS316 tube having an outer diameter of 1/16 inches, an inner diameter of 1.0 mm, and a length of 2.0 m, and the joining portions J2 and J4 were T-shaped connectors having an inner diameter of 2.3 mm.

The results are shown in the table below.

**[Table 3]**

| | Moisture content (ppm) | First stage reaction | | Second stage reaction | | LC production rate (%) |
|---|---|---|---|---|---|---|
| | | Organometallic compound (molar equivalent) | Electrophilic compound (molar equivalent) | Organometallic compound (molar equivalent) | Electrophilic compound (molar equivalent) | |
| Example 1 | 184 | 1.0 | 1.0 | 1.0 | 1.5 | 63 |
| Example 2 | 78 | 1.0 | 1.0 | 1.0 | 1.5 | 64 |
| Example 3 | 184 | 1.0 | 1.0 | 1.0 | 1.2 | 61 |
| Example 4 | 78 | 1.0 | 1.0 | 1.0 | 1.2 | 63 |
| Example 5 | 184 | 1.0 | 1.0 | 1.0 | 1.0 | 60 |
| Example 6 | 78 | 1.0 | 1.0 | 1.0 | 1.0 | 63 |
| Example 7 | 59 | 1.0 | 1.0 | 1.0 | 1.0 | 65 |
| Example 8 | 11 | 1.0 | 1.0 | 1.0 | 1.0 | 66 |
| Example 9 | 184 | 1.0 | 1.0 | 1.0 | 0.8 | 48 |
| Example 10 | 78 | 1.0 | 1.0 | 1.0 | 0.8 | 51 |
| Example 11 | 77 | 1.0 | 1.0 | 1.0 | 1.0 | 61 |
| Example 12 | 89 | 1.0 | 1.0 | 1.0 | 1.0 | 64 |
| Example 13 | 72 | 1.0 | 1.0 | 1.0 | 1.0 | 63 |
| Example 14 | 59 | 1.0 | 1.0 | 1.0 | 1.0 | 61 |
| Example 15 | 74 | 1.0 | 1.0 | 1.0 | 1.0 | 64 |
| Example 16 | 59 | 1.0 | 1.0 | 1.0 | 1.0 | 64 |
| Example 17 | 42 | 1.0 | 1.0 | 1.1 | 1.0 | 65 |
| Example 18 | 7 | 0.9 | 1.0 | 1.0 | 1.0 | 62 |
| Example 19 | 6 | 1.0 | 1.0 | 1.0 | 1.0 | 66 |
| Example 20 | 6 | 1.0 | 1.0 | 1.0 | 1.0 | 65 |
| Example 21 | 6 | 1.0 | 1.0 | 1.0 | 1.0 | 65 |
| Example 22 | 6 | 0.9 | 0.9 | 0.9 | 0.9 | 56 |
| Example 23 | 6 | 0.9 | 0.9 | 0.9 | 0.9 | 61 |
| Example 24 | 6 | 0.9 | 0.9 | 0.9 | 0.9 | 63 |

As shown in the above table, by reducing the moisture content of each solution to be allowed to flow in the flow channel to the range specified in the present invention, the desired compound could be obtained at a sufficiently high production rate even in a case where the amount of the electrophilic compound used was reduced.

### [Reference Example 2]

Using the flow-type reaction system 100 having the configuration shown in FIG. 1, a desired electrophilic substituted aromatic compound (desired compound) was prepared in the same manner as in Reference Example 1. In this regard, the moisture content of each solution to be allowed to flow in the flow channel was adjusted as shown in the table below, the temperatures of the flow channels 1, 2, and 3, the joining portions J1 and J2, and the reaction tubes F1 and F2 were set to 10°C, and a solution obtained by dissolving 1,5-dibromonaphthalene in THF (1,5-dibromonaphthalene solution, 1,5-dibromonaphthalene concentration: 0.35 M) was used as the liquid A1 containing a dihalogenated aromatic compound in an organic solvent. In addition, the joining portion J5 was a T-shaped mixer having an inner diameter of 2.3 mm.

Identification of the desired compound was carried out by 1H-NMR.

Chemical shifts σ (ppm) by ¹H-NMR (400 MHz, solvent: CDCl₃, internal reference substance: tetramethylsilane (TMS)) = 8.90-8.88 (2H, m), 8.43-8.40 (6H, m), 8.37-8.35 (2H, m), 8.19-8.17 (2H, m), 8.09-8.04 (4H, m), 7.91-7.87 (4H, m), 7.73-7.70 (2H, m), 7.53-7.49 (4H, m), 7.45-7.41 (2H, m)

The results are shown in the table below. In the following table, the molar equivalent is a molar amount with respect to 1 mole of a halogenated aromatic compound as a raw material.

**[Table 4]**

| | Moisture content (ppm) | First stage reaction | | Second stage reaction | | LC production rate (%) |
|---|---|---|---|---|---|---|
| | | Organometallic compound (molar equivalent) | Electrophilic compound (molar equivalent) | Organometallic compound (molar equivalent) | Electrophilic compound (molar equivalent) | |
| Reference Example 2 | 247 | 1.0 | 1.0 | 1.0 | 2.0 | 72 |

It was confirmed that, in the final stage (second stage) reaction, in a case where an excess amount of the electrophilic compound was used, the production rate of the desired compound was increased even in a case where the moisture content of each solution flowing in the flow channel was higher (247 ppm) than that specified in the present invention.

### [Comparative Examples 5 to 7]

The desired electrophilic substituted aromatic compound was prepared in the same manner as in Reference Example 2, except that, in Reference Example 2, the amount of the electrophilic compound used in the final stage (second stage) reaction was reduced as shown in the table below by adjusting the flow velocity of the 2-phenyl-1,10-phenanthroline solution C2.

The results are shown in the table below.

**[Table 5]**

| | Moisture content (ppm) | First stage reaction | | Second stage reaction | | LC production rate (%) |
|---|---|---|---|---|---|---|
| | | Organometallic compound (molar equivalent) | Electrophilic compound (molar equivalent) | Organometallic compound (molar equivalent) | Electrophilic compound (molar equivalent) | |
| Comparative Example 5 | 247 | 1.0 | 1.0 | 1.0 | 1.2 | 62 |
| Comparative Example 6 | 247 | 1.0 | 1.0 | 1.0 | 1.0 | 56 |
| Comparative Example 7 | 247 | 1.0 | 1.0 | 1.0 | 0.8 | 41 |

It can be seen that, in the final stage (second stage) reaction, in a case where the amount of the electrophilic compound used is smaller than that in Reference Example 2, and then in a case where the moisture content of each solution flowing in the flow channel is higher (247 ppm) than that specified in the present invention, the production rate of the desired compound decreases depending on the amount of the electrophilic compound used.

### [Examples 25 to 33]

A desired electrophilic substituted aromatic compound was prepared in the same manner as in Reference Example 2, except that the moisture content of each solution to be allowed to flow in the flow channel was set as shown in the table which will be given later and the amount of the electrophilic compound used in the final stage (second stage) reaction was adjusted by adjusting the flow velocity of the 2-phenyl-1,10-phenanthroline solution C2.

### [Example 34]

A desired electrophilic substituted aromatic compound was prepared in the same manner as in Example 28, except that the moisture content of each solution to be allowed to flow in the flow channel was set as shown in the table which will be given later, the temperatures of the flow channels 1 and 2, the joining portion J1, and the reaction tube F1 were set to 0°C, and the temperatures of the flow channel 3, the joining portion J2, and the reaction tube F2 were set to 20°C.

### [Example 35]

A desired electrophilic substituted aromatic compound was prepared in the same manner as in Example 34, except that the moisture content of each solution to be allowed to flow in the flow channel was set as shown in the table which will be given later, the length of the reaction tube F1 was set to 30 cm, the length of the reaction tube F2 to the T-shaped connector was set to 1.2 m, the length of the reaction tube F3 was set to 1.0 m, the length of the reaction tube F4 was set to 4.0 m, and the temperatures of the flow channel 4, the joining portion J3, and the reaction tube F3 were set to 0°C.

### [Example 36]

A desired electrophilic substituted aromatic compound was prepared in the same manner as in Example 35, except that the moisture content of each solution to be allowed to flow in the flow channel was set as shown in the table which will be given later.

### [Example 37]

A desired electrophilic substituted aromatic compound was prepared in the same manner as in Example 36, except that the moisture content of each solution to be allowed to flow in the flow channel was set as shown in the table which will be given later and the temperatures of the flow channels 1 to 6, the joining portions J1 to J5, and the reaction tubes F1 to F5 were set to 10°C.

### [Example 38]

A desired electrophilic substituted aromatic compound was prepared in the same manner as in Example 28, except that the moisture content of each solution to be allowed to flow in the flow channel was set as shown in the table which will be given later, the temperatures of the flow channel 3, the joining portion J2, and the reaction tube F2 were set to 50°C, and the temperatures of the flow channel 5, the joining portion J4, and the reaction tube F4 were set to 20°C.

### [Example 39]

A desired electrophilic substituted aromatic compound was prepared in the same manner as in Example 28, except that the moisture content of each solution to be allowed to flow in the flow channel was set as shown in the table which will be given later, the flow channel 1 was an SUS316 tube having an outer diameter of 1/16 inches, an inner diameter of 1.0 mm, and a length of 50 cm, the reaction tube F2 was an SUS316 tube having an outer diameter of 1/16 inches, an inner diameter of 1.0 mm, and a length of 2.0 m, and the joining portions J1 and J3 were T-shaped connectors (inner diameter: 1.0 mm).

The results are shown in the table below.

**[Table 6]**

| | Moisture content (ppm) | First stage reaction | | Second stage reaction | | LC production rate (%) |
|---|---|---|---|---|---|---|
| | | Organometallic compound (molar equivalent) | Electrophilic compound (molar equivalent) | Organometallic compound (molar equivalent) | Electrophilic compound (molar equivalent) | |
| Example 25 | 176 | 1.0 | 1.0 | 1.0 | 1.2 | 69 |
| Example 26 | 91 | 1.0 | 1.0 | 1.0 | 1.2 | 73 |
| Example 27 | 12 | 1.0 | 1.0 | 1.0 | 1.2 | 75 |
| Example 28 | 176 | 1.0 | 1.0 | 1.0 | 1.0 | 67 |
| Example 29 | 91 | 1.0 | 1.0 | 1.0 | 1.0 | 71 |
| Example 30 | 12 | 1.0 | 1.0 | 1.0 | 1.0 | 74 |
| Example 31 | 176 | 1.0 | 1.0 | 1.0 | 0.8 | 54 |
| Example 32 | 91 | 1.0 | 1.0 | 1.0 | 0.8 | 58 |
| Example 33 | 12 | 1.0 | 1.0 | 1.0 | 0.8 | 63 |
| Example 34 | 22 | 1.0 | 1.0 | 1.0 | 1.0 | 70 |
| Example 35 | 48 | 1.0 | 1.0 | 1.0 | 1.0 | 71 |
| Example 36 | 31 | 1.0 | 1.0 | 1.0 | 1.0 | 70 |
| Example 37 | 19 | 1.0 | 1.0 | 1.0 | 1.0 | 66 |
| Example 38 | 21 | 1.0 | 1.0 | 1.0 | 1.0 | 72 |
| Example 39 | 12 | 1.0 | 1.0 | 1.0 | 1.0 | 73 |

As shown in the above table, by reducing the moisture content of each solution to be allowed to flow in the flow channel to the range specified in the present invention, the desired compound could be obtained at a sufficiently high production rate even in a case where the amount of the electrophilic compound used was reduced.

### [Reference Example 3]

A desired electrophilic substituted aromatic compound (desired compound) was prepared using the flow-type reaction system 100 having the configuration shown in FIG. 1. The desired electrophilic substituted aromatic compound was prepared in the same manner as in Reference Example 1, except that the moisture content of each solution to be allowed to flow in the flow channel was set as shown in the table which will be given later and the following reaction conditions were used. Specific reaction conditions are as follows.

### Temperature control:

The temperatures of the flow channels 1 and 2, the joining portion J1, and the reaction tube F1 were set to 0°C.

The temperatures of the flow channel 3, the joining portion J2, and the reaction tube F2 were set to 50°C.

The temperatures of the flow channel 4, the joining portion J3, and the reaction tube F3 were set to -20°C.

The temperatures of the flow channel 5, the joining portion J4, and the reaction tube F4 were set to 5°C.

The temperatures of the flow channel 6, the joining portion J5, and the reaction tube F5 were set to 5°C.

### Reaction tubes F1 to F4:

In all of the reaction tubes F1 to F4, an SUS316 tube having an outer diameter of 1/16 inches and an inner diameter of 1.0 mm was used. The length of each flow channel was as follows.
Reaction tube F1: 0.8 m
Reaction tube F2: 1.0 m
Reaction tube F3: 1.0 m
Reaction tube F4: 1.0 m

### Reaction tube F5:

A PTFE tube having an outer diameter of 1/8 inches, an inner diameter of 1.6 mm, and a length of 1.5 m was used.

### Joining portion J5:

A T-shaped connector having an inner diameter of 2.3 mm was used.

### Liquid A1 containing dihalogenated aromatic compound in organic solvent:

A solution obtained by dissolving 1,3-dibromobenzene in THF (1,3-dibromobenzene solution A1, 1,3-dibromobenzene concentration: 0.34 M) was prepared.

### Liquid B1 containing organometallic compound in organic solvent:

n-BuLi (n-butyl lithium solution B1, n-BuLi concentration: 1.6 M, n-hexane solution) manufactured by Kanto Chemical Co., Inc. was used.

### Liquid B2 containing organometallic compound in organic solvent:

n-BuLi (n-butyl lithium solution B2, n-BuLi concentration: 1.6 M, n-hexane solution) manufactured by Kanto Chemical Co., Inc. was used.

### Liquid C1 containing electrophilic compound in organic solvent:

A solution obtained by dissolving 2-phenyl-1,10-phenanthroline in THF (2-phenyl-1,10-phenanthroline solution C1, 2-phenyl-1,10-phenanthroline concentration: 0.27 M) was prepared and used in the first stage reaction.

### Liquid C2 containing electrophilic compound in organic solvent:

A solution obtained by dissolving 2-phenyl-1,10-phenanthroline in THF (2-phenyl-1,10-phenanthroline solution C2, 2-phenyl-1,10-phenanthroline concentration: 0.27 M) was prepared and used in the second stage reaction.

### Liquid feeding conditions:

1,3-Dibromobenzene solution: 5.1 mL/min
n-Butyl lithium solution B1: 1.1 mL/min
n-Butyl lithium solution B2: 1.1 mL/min
2-Phenyl-1,10-phenanthroline solution C1: 6.4 mL/min
2-Phenyl-1,10-phenanthroline solution C2: 12.8 mL/min
Water (X): 6.9 mL/min
Identification of the desired compound was carried out by 1H-NMR.
Chemical shifts σ (ppm) by ¹H-NMR (400 MHz, solvent: DMSO-d6, internal reference substance: tetramethylsilane (TMS)) = 9.84 (1H, m), 8.74-8.72 (4H, m), 8.67-8.60 (4H, m), 8.54-8.52 (4H, m), 8.41-8.39 (2H, m), 8.13-8.07 (4H, m), 7.94-7.90 (1H, m), 7.46-7.40 (6H, m)

The results are shown in the table below. In the following table, the molar equivalent is a molar amount with respect to 1 mole of a halogenated aromatic compound as a raw material.

**[Table 7]**

| | Moisture content (ppm) | First stage reaction | | Second stage reaction | | LC production rate (%) |
|---|---|---|---|---|---|---|
| | | Organometallic compound (molar equivalent) | Electrophilic compound (molar equivalent) | Organometallic compound (molar equivalent) | Electrophilic compound (molar equivalent) | |
| Reference Example 3 | 258 | 1.0 | 1.0 | 1.0 | 2.0 | 78 |

It was confirmed that, in the final stage (second stage) reaction, in a case where an excess amount of the electrophilic compound was used, the production rate of the desired compound was increased even in a case where the moisture content of each solution flowing in the flow channel was higher (258 ppm) than that specified in the present invention.

### [Comparative Examples 8 and 9]

The desired electrophilic substituted aromatic compound was prepared in the same manner as in Reference Example 3, except that, in Reference Example 3, the amount of the electrophilic compound used in the final stage (second stage) reaction was reduced as shown in the table below by adjusting the flow velocity of the 2-phenyl-1,10-phenanthroline solution C2.

The results are shown in the table below.

**[Table 8]**

| | Moisture content (ppm) | First stage reaction | | Second stage reaction | | LC production rate (%) |
|---|---|---|---|---|---|---|
| | | Organometallic compound (molar equivalent) | Electrophilic compound (molar equivalent) | Organometallic compound (molar equivalent) | Electrophilic compound (molar equivalent) | |
| Comparative Example 8 | 258 | 1.0 | 1.0 | 1.0 | 1.0 | 59 |
| Comparative Example 9 | 258 | 1.0 | 1.0 | 1.0 | 0.8 | 44 |

It can be seen that, in the final stage (second stage) reaction, in a case where the amount of the electrophilic compound used is smaller than that in Reference Example 3, and then in a case where the moisture content of each solution flowing in the flow channel is higher (258 ppm) than that specified in the present invention, the production rate of the desired compound decreases depending on the amount of the electrophilic compound used.

### [Examples 40 and 41]

A desired electrophilic substituted aromatic compound was prepared in the same manner as in Reference Example 3, except that the moisture content of each solution to be allowed to flow in the flow channel was set as shown in the table which will be given later and the amount of the electrophilic compound used in the final stage (second stage) reaction was adjusted by adjusting the flow velocity of the 2-phenyl-1,10-phenanthroline solution C2.

The results are shown in the table below.

**[Table 9]**

| | Moisture content (ppm) | First stage reaction | | Second stage reaction | | LC production rate (%) |
|---|---|---|---|---|---|---|
| | | Organometallic compound (molar equivalent) | Electrophilic compound (molar equivalent) | Organometallic compound (molar equivalent) | Electrophilic compound (molar equivalent) | |
| Example 40 | 8 | 1.0 | 1.0 | 1.0 | 1.0 | 80 |
| Example 41 | 8 | 1.0 | 1.0 | 1.0 | 0.8 | 63 |

As shown in the above table, by reducing the moisture content of each solution to be allowed to flow in the flow channel to the range specified in the present invention, the desired compound could be obtained at a sufficiently high production rate even in a case where the amount of the electrophilic compound used was reduced.

### [Reference Example 4]

A desired electrophilic substituted aromatic compound (desired compound) was prepared using the flow-type reaction system 200 having the configuration shown in FIG. 4. The desired electrophilic substituted aromatic compound was prepared in the same manner as in Reference Example 1, except that the moisture content of each solution to be allowed to flow in the flow channel was set as shown in the table which will be given later and the following reaction conditions were used. Specific reaction conditions are as follows.

### Temperature control:

The temperatures of the flow channels 7 and 8, the joining portion J6, and the reaction tube F6 were set to 10°C.

The temperatures of the flow channel 9, the joining portion J7, and the reaction tube F7 were set to 50°C.

The temperatures of the flow channel 10, the joining portion J8, and the reaction tube F8 were set to 10°C.

### Flow channels 7 to 10:

In all of the flow channels 7 to 10, an SUS316 tube having an outer diameter of 1/16 inches, an inner diameter of 1.0 mm, and a length of 50 cm was used.

### Reaction tubes F6 and F7:

In both of the reaction tubes F6 and F7, an SUS316 tube having an outer diameter of 1/16 inches and an inner diameter of 1.0 mm was used. The length of each flow channel was as follows.
Reaction tube F6: 3.0 m
Reaction tube F7: 1.0 m

### Reaction tube F8:

A PTFE tube having an outer diameter of 1/8 inches, an inner diameter of 1.6 mm, and a length of 1.5 m was used.

### Joining portion J6:

A T-shaped connector having an inner diameter of 1.3 mm was used. The flow channel 7 and the flow channel 8 were respectively connected to two connection ports facing each other among three connection ports of the T-shaped connector. The remaining connection port was used as a discharge port for discharging the liquid.

### Joining portion J7:

A T-shaped connector having an inner diameter of 1.3 mm was used. The reaction tube F6 and the flow channel 9 were respectively connected to two connection ports facing each other among three connection ports of the T-shaped connector. The remaining connection port was used as a discharge port for discharging the liquid.

### Joining portion J8:

A T-shaped connector having an inner diameter of 2.3 mm was used. The reaction tube F7 and the flow channel 10 were respectively connected to two connection ports facing each other among three connection ports of the T-shaped connector. The remaining connection port was used as a discharge port for discharging the liquid.

### Liquid A2 containing halogenated aromatic compound in organic solvent:

A solution obtained by dissolving bromobenzene in THF (bromobenzene solution, bromobenzene concentration: 3.2 M) was prepared.

### Liquid B3 containing organometallic compound in organic solvent:

n-BuLi (n-butyl lithium solution B3, n-BuLi concentration: 1.6 M, n-hexane solution) manufactured by Kanto Chemical Co., Inc. was used.

### Liquid C3 containing electrophilic compound in organic solvent:

A solution obtained by dissolving 1,10-phenanthroline in THF (1,10-phenanthroline solution C3, 1,10-phenanthroline concentration: 0.89 M) was prepared.

### Liquid feeding conditions:

Bromobenzene solution A2: 1.4 mL/min
n-Butyl lithium solution B3: 2.8 mL/min
1,10-Phenanthroline solution C3: 6.0 mL/min
Water (X): 4.0 mL/min
Identification of the desired compound was carried out by 1H-NMR.
Chemical shifts σ (ppm) by ¹H-NMR (400 MHz, solvent: DMSO-d6, internal reference substance: tetramethylsilane (TMS)) = 9.18-9.17 (1H, m), 8.60-8.39 (5H, m), 8.05-7.98 (2H, m), 7.82-7.79 (1H, m), 7.64-7.54 (3H, m)

The results are shown in the table below. In the following table, the molar equivalent is a molar amount with respect to 1 mole of a halogenated aromatic compound as a raw material.

**[Table 10]**

| | Moisture content (ppm) | First stage reaction | | LC production rate (%) |
|---|---|---|---|---|
| | | Organometallic compound (molar equivalent) | Electrophilic compound (molar equivalent) | |
| Reference Example 4 | 316 | 1.0 | 1.2 | 91 |

It was confirmed that, in a case where an excess amount of the electrophilic compound was used, the production rate of the desired compound was increased even in a case where the moisture content of each solution flowing in the flow channel was higher (316 ppm) than that specified in the present invention.

### [Comparative Examples 10 and 11]

A desired electrophilic substituted aromatic compound was prepared in the same manner as in Reference Example 4, except that, in Reference Example 4, the amount of the electrophilic compound used in the reaction was reduced as shown in the table below by adjusting the flow velocity of the 1,10-phenanthroline solution C3.

The results are shown in the table below.

**[Table 11]**

| | Moisture content (ppm) | First stage reaction | | LC production rate (%) |
|---|---|---|---|---|
| | | Organometallic compound (molar equivalent) | Electrophilic compound (molar equivalent) | |
| Comparative Example 10 | 316 | 1.0 | 1.0 | 82 |
| Comparative Example 11 | 316 | 1.0 | 0.8 | 60 |

It can be seen that, in a case where the amount of the electrophilic compound used is smaller than that in Reference Example 4, and then in a case where the moisture content of each solution flowing in the flow channel is higher (316 ppm) than that specified in the present invention, the production rate of the desired compound decreases depending on the amount of the electrophilic compound used.

### [Examples 42 to 45]

A desired electrophilic substituted aromatic compound was prepared in the same manner as in Reference Example 4, except that the moisture content of each solution to be allowed to flow in the flow channel was set as shown in the table which will be given later and the amount of the electrophilic compound used in the reaction was adjusted by adjusting the flow velocity of the 1,10-phenanthroline solution C3.

### [Example 46]

A desired electrophilic substituted aromatic compound was prepared in the same manner as in Example 42, except that each solution to be allowed to flow in the flow channel was passed through a dehydration column disposed in the flow channel to adjust the moisture content.

### [Example 47]

A desired electrophilic substituted aromatic compound was prepared in the same manner as in Example 46, except that each solution to be allowed to flow in the flow channel was passed through a dehydration column disposed in the flow channel to adjust the moisture content, and a quenching treatment was carried out in a batch manner.

### [Example 48]

A desired electrophilic substituted aromatic compound was prepared in the same manner as in Example 46, except that each solution to be allowed to flow in the flow channel was passed through a dehydration column disposed in the flow channel to adjust the moisture content, the temperatures of the flow channels 7 and 8, the joining portion J6, and the reaction tube F6 were set to 30°C, and the length of the reaction tube F6 was set to 1.0 m.

### [Example 49]

A desired electrophilic substituted aromatic compound was prepared in the same manner as in Example 46, except that each solution to be allowed to flow in the flow channel was passed through a dehydration column disposed in the flow channel to adjust the moisture content, and the amount of the organometallic compound and the amount of the electrophilic compound used in the reaction were adjusted by adjusting the flow velocities of the n-BuLi solution B3 and the 1,10-phenanthroline solution C3.

The results are shown in the table below.

**[Table 12]**

| | Moisture content (ppm) | First stage reaction | | LC production rate (%) |
|---|---|---|---|---|
| | | Organometallic compound (molar equivalent) | Electrophilic compound (molar equivalent) | |
| Example 42 | 168 | 1.0 | 1.0 | 90 |
| Example 43 | 27 | 1.0 | 1.0 | 93 |
| Example 44 | 168 | 1.0 | 0.8 | 68 |
| Example 45 | 21 | 1.0 | 0.8 | 72 |
| Example 46 | 21 | 1.0 | 1.0 | 92 |
| Example 47 | 21 | 1.0 | 1.0 | 93 |
| Example 48 | 21 | 1.0 | 1.0 | 91 |
| Example 49 | 21 | 0.8 | 0.8 | 76 |

As shown in the above table, by reducing the moisture content of each solution to be allowed to flow in the flow channel to the range specified in the present invention, the desired compound could be obtained at a sufficiently high production rate even in a case where the amount of the electrophilic compound used was reduced.

### [Comparative Example 12]

A desired electrophilic substituted aromatic compound (desired compound) was prepared using the flow-type reaction system 100 having the configuration shown in FIG. 1. The desired electrophilic substituted aromatic compound was prepared in the same manner as in Reference Example 1, except that the moisture content of each solution to be allowed to flow in the flow channel was adjusted and the following reaction conditions were used. Specific reaction conditions are as follows.

### Temperature control:

The temperatures of the flow channels 1 and 2, the joining portion J1, and the reaction tube F1 were set to 0°C.

The temperatures of the flow channel 3, the joining portion J2, and the reaction tube F2 were set to 0°C.

The temperatures of the flow channel 4, the joining portion J3, and the reaction tube F3 were set to -20°C.

The temperatures of the flow channel 5, the joining portion J4, and the reaction tube F4 were set to 5°C.

The temperatures of the flow channel 6, the joining portion J5, and the reaction tube F5 were set to 5°C.

### Flow channels 1 to 6:

In all of the flow channels 1 to 6, an SUS316 tube having an outer diameter of 1/16 inches, an inner diameter of 1.0 mm, and a length of 50 cm was used.

### Reaction tubes F1 to F4:

In all of the reaction tubes F1 to F4, an SUS316 tube having an outer diameter of 1/16 inches and an inner diameter of 1.0 mm was used. The length of each flow channel was as follows.
Reaction tube F1: 0.5 m
Reaction tube F2: 1.5 m
Reaction tube F3: 2.0 m
Reaction tube F4: 1.5 m

### Reaction tube F5:

A PTFE tube having an outer diameter of 1/8 inches, an inner diameter of 1.6 mm, and a length of 1.0 m was used.

### Joining portion J1:

A T-shaped connector having an inner diameter of 1.0 mm was used. The flow channel 1 and the flow channel 2 were respectively connected to two connection ports facing each other among three connection ports of the T-shaped connector. The remaining connection port was used as a discharge port for discharging the liquid.

### Joining portion J2:

A T-shaped connector having an inner diameter of 1.0 mm was used. The reaction tube F1 and the flow channel 3 were respectively connected to two connection ports facing each other among three connection ports of the T-shaped connector. The remaining connection port was used as a discharge port for discharging the liquid.

### Joining portion J3:

A T-shaped connector having an inner diameter of 1.0 mm was used. The reaction tube F2 and the flow channel 4 were respectively connected to two connection ports facing each other among three connection ports of the T-shaped connector. The remaining connection port was used as a discharge port for discharging the liquid.

### Joining portion J4:

A T-shaped connector having an inner diameter of 1.0 mm was used. The reaction tube F3 and the flow channel 5 were respectively connected to two connection ports facing each other among three connection ports of the T-shaped connector. The remaining connection port was used as a discharge port for discharging the liquid.

### Joining portion J5:

A T-shaped connector having an inner diameter of 2.3 mm was used. The reaction tube F4 and the flow channel 6 were respectively connected to two connection ports facing each other among three connection ports of the T-shaped connector. The remaining connection port was used as a discharge port for discharging the liquid.

### Liquid A1 containing dihalogenated aromatic compound in organic solvent:

A solution obtained by dissolving 1,4-dibromobenzene in THF (1,4-dibromobenzene solution, 1,4-dibromobenzene concentration: 0.34 M) was prepared.

### Liquid B1 containing organometallic compound in organic solvent:

n-BuLi (n-butyl lithium solution B1, n-BuLi concentration: 1.6 M, n-hexane solution) manufactured by Kanto Chemical Co., Inc. was used.

### Liquid B2 containing organometallic compound in organic solvent:

n-BuLi (n-butyl lithium solution B2, n-BuLi concentration: 1.6 M, n-hexane solution) manufactured by Kanto Chemical Co., Inc. was used.

### Liquid C1 containing electrophilic compound in organic solvent:

A solution obtained by dissolving chlorotrimethylsilane in THF (chlorotrimethylsilane solution C1, chlorotrimethylsilane concentration: 0.23 M) was prepared and used in the first stage reaction.

### Liquid C2 containing electrophilic compound in organic solvent:

A solution obtained by dissolving benzaldehyde in THF (benzaldehyde solution C2, benzaldehyde concentration: 0.24 M) was prepared and used in the second stage reaction.

### Liquid feeding conditions:

1,4-Dibromobenzene solution A1: 4.1 mL/min
n-Butyl lithium solution B1: 0.9 mL/min
n-Butyl lithium solution B2: 0.9 mL/min
Chlorotrimethylsilane solution C1: 6.0 mL/min
Benzaldehyde solution C2: 7.1 mL/min
Water (X): 1.5 mL/min

### [Comparative Example 13]

A desired electrophilic substituted aromatic compound was prepared in the same manner as in Comparative Example 12, except that the amount of the electrophilic compound used in the reaction was reduced as shown in the table below by adjusting the flow velocity of the benzaldehyde solution C2.

### [Examples 50 and 51]

A desired electrophilic substituted aromatic compound was prepared in the same manner as in Comparative Example 12, except that the moisture content of each solution to be allowed to flow in the flow channel was set as shown in the table below and the amount of the electrophilic compound used in the reaction was prepared as shown in the table below by adjusting the flow velocity of the benzaldehyde solution C2.

### The results are shown in the table below.

**[Table 13]**

| | Moisture content (ppm) | First stage reaction | | Second stage reaction | | GC production rate (%) |
|---|---|---|---|---|---|---|
| | | Organometallic compound (molar equivalent) | Electrophilic compound (molar equivalent) | Organometallic compound (molar equivalent) | Electrophilic compound (molar equivalent) | |
| Comparative Example 12 | 323 | 1.0 | 1.0 | 1.0 | 1.2 | 66 |
| Comparative Example 13 | 323 | 1.0 | 1.0 | 1.0 | 1.0 | 63 |
| Example 50 | 33 | 1.0 | 1.0 | 1.0 | 1.2 | 77 |
| Example 51 | 33 | 1.0 | 1.0 | 1.0 | 1.0 | 74 |

### [Comparative Example 14]

A desired electrophilic substituted aromatic compound (desired compound) was prepared using the flow-type reaction system 100 having the configuration shown in FIG. 1. The desired electrophilic substituted aromatic compound was prepared in the same manner as in Comparative Example 12, except that the moisture content of each solution to be allowed to flow in the flow channel was set as shown in the table which will be given later and the following reaction conditions were used. Specific reaction conditions are as follows.

### Liquid A1 containing dihalogenated aromatic compound in organic solvent:

A solution obtained by dissolving 1,3-dibromobenzene in THF (1,3-dibromobenzene solution, 1,3-dibromobenzene concentration: 0.68 M) was prepared.

### Liquid B1 containing organometallic compound in organic solvent:

n-BuLi (n-butyl lithium solution B1, n-BuLi concentration: 1.6 M, n-hexane solution) manufactured by Kanto Chemical Co., Inc. was used.

### Liquid B2 containing organometallic compound in organic solvent:

n-BuLi (n-butyl lithium solution B2, n-BuLi concentration: 1.6 M, n-hexane solution) manufactured by Kanto Chemical Co., Inc. was used.

### Liquid C1 containing electrophilic compound in organic solvent:

A solution obtained by dissolving chlorotrimethylsilane in THF (chlorotrimethylsilane solution C1, chlorotrimethylsilane concentration: 0.23 M) was prepared and used in the first stage reaction.

### Liquid C2 containing electrophilic compound in organic solvent:

A solution obtained by dissolving chlorotrimethylsilane in THF (chlorotrimethylsilane solution C2, chlorotrimethylsilane concentration: 0.23 M) was prepared and used in the second stage reaction.

### Liquid feeding conditions:

1,3-Dibromobenzene solution A1: 2.0 mL/min
n-Butyl lithium solution B1: 0.9 mL/min
n-Butyl lithium solution B2: 0.9 mL/min
Chlorotrimethylsilane solution C1: 6.0 mL/min
Chlorotrimethylsilane solution C2: 7.2 mL/min
Water (X): 1.5 mL/min

### [Comparative Example 15]

A desired electrophilic substituted aromatic compound was prepared in the same manner as in Comparative Example 14, except that the amount of the electrophilic compound used in the reaction was reduced as shown in the table which will be given later by adjusting the flow velocity of the chlorotrimethylsilane solution C2.

### [Examples 52 and 53]

A desired electrophilic substituted aromatic compound was prepared in the same manner as in Comparative Example 14, except that the moisture content of each solution to be allowed to flow in the flow channel was adjusted, and the amount of the electrophilic compound used in the reaction was prepared as shown in the table below by adjusting the flow velocity of the chlorotrimethylsilane solution C2.

The results are shown in the table below.

**[Table 14]**

| | Moisture content (ppm) | First stage reaction | | Second stage reaction | | GC production rate (%) |
|---|---|---|---|---|---|---|
| | | Organometallic compound (molar equivalent) | Electrophilic compound (molar equivalent) | Organometallic compound (molar equivalent) | Electrophilic compound (molar equivalent) | |
| Comparative Example 14 | 251 | 1.0 | 1.0 | 1.0 | 1.2 | 56 |
| Comparative Example 15 | 251 | 1.0 | 1.0 | 1.0 | 1.0 | 51 |
| Example 52 | 9 | 1.0 | 1.0 | 1.0 | 1.2 | 60 |
| Example 53 | 9 | 1.0 | 1.0 | 1.0 | 1.0 | 57 |

### [Comparative Example 16]

According to the reaction scheme shown in FIG. 7, a desired electrophilic substituted aromatic compound (desired compound) was prepared using the flow-type reaction system 400 having the configuration shown in FIG. 6. The desired electrophilic substituted aromatic compound was prepared under the following reaction conditions, in which the moisture content of each solution to be allowed to flow in the flow channel was set as shown in the table which will be given later. Specific reaction conditions are as follows.

### Temperature control:

The temperatures of the flow channels 17 and 18, the joining portion J14, and the reaction tube F14 were set to 10°C.

The temperatures of the flow channel 19, the joining portion J15, and the reaction tube F15 were set to 10°C.

The temperatures of the flow channel 20, the joining portion J16, and the reaction tube F16 were set to 50°C.

The temperatures of the flow channel 21, the joining portion J17, and the reaction tube F17 were set to 5°C.

### Flow channel 17, flow channel 18, flow channel 19, flow channel 20, and flow channel 21:

An SUS316 tube having an outer diameter of 1/16 inches, an inner diameter of 1.0 mm, and a length of 50 cm was used.

### Reaction tube F14:

An SUS316 tube having an outer diameter of 1/16 inches, an inner diameter of 1.0 mm, and a length of 1.5 m was used.

### Reaction tube F15:

The reaction tube F15 had a structure established by dividing a single SUS tube into two parts by using a T-shaped connector. More specifically, a T-shaped connector (inner diameter: 1.0 mm) was connected to an SUS316 tube having an outer diameter of 1/16 inches, an inner diameter of 1.0 mm, and a length of 1.0 m, and two SUS316 tubes having an outer diameter of 1/16 inches, an inner diameter of 1.0 mm, and a length of 3 cm were connected to the T-shaped connector to face each other, thus forming a reaction tube F15.

### Reaction tube F16:

An SUS316 tube having an outer diameter of 1/16 inches, an inner diameter of 1.0 mm, and a length of 3.0 m was used.

### Reaction tube F17:

A PTFE tube having an outer diameter of 1/8 inches, an inner diameter of 1.6 mm, and a length of 1.5 m was used.

### Joining portion J14:

A T-shaped connector having an inner diameter of 1.0 mm was used. The flow channel 17 and the flow channel 18 were respectively connected to two connection ports facing each other among three connection ports of the T-shaped connector. The remaining connection port was used as a discharge port for discharging the liquid.

### Joining portion J15:

A T-shaped connector having an inner diameter of 1.0 mm was used. The reaction tube F14 and the flow channel 19 were respectively connected to two connection ports facing each other among three connection ports of the T-shaped connector. The remaining connection port was used as a discharge port for discharging the liquid.

### Joining portion J16:

A cross connector having an inner diameter of 1.0 mm was used. Two SUS316 tubes connected to the T-shaped connector constituting the reaction tube F15 to face each other were respectively connected to two connection ports facing each other among four connection ports of the cross connector. The flow channel 20 was connected to one of the remaining two connection ports and the remaining connection port was used as a discharge port for discharging the liquid.

### Joining portion J17:

A T-shaped connector having an inner diameter of 2.3 mm was used. The reaction tube F16 and the flow channel 21 were respectively connected to two connection ports facing each other among three connection ports of the T-shaped connector. The remaining connection port was used as a discharge port for discharging the liquid.

### Liquid A5 containing halogenated aromatic compound in organic solvent:

A solution obtained by dissolving bromobenzene in THF (bromobenzene solution, bromobenzene concentration: 0.64 M) was prepared.

### Liquid B6 containing organometallic compound in organic solvent:

n-BuLi (n-butyl lithium solution B6, n-BuLi concentration: 1.6 M, n-hexane solution) manufactured by Kanto Chemical Co., Inc. was used.

### Liquid C5 containing electrophilic compound in organic solvent:

A solution obtained by dissolving 1,10-phenanthroline in THF (1,10-phenanthroline solution C5, 1,10-phenanthroline concentration: 0.55 M) was prepared.

### Liquid D containing nucleophilic compound in organic solvent:

n-BuLi (n-butyl lithium solution D, n-BuLi concentration: 1.6 M, n-hexane solution) manufactured by Kanto Chemical Co., Inc. was used.

### Liquid feeding conditions:

Bromobenzene solution A5: 5.2 mL/min
n-Butyl lithium solution B6: 2.1 mL/min
1,10-Phenanthroline solution C5: 6.0 mL/min
n-Butyl lithium solution D: 2.1 mL/min
Water (X): 3.0 mL/min

### [Comparative Example 17]

A desired electrophilic substituted aromatic compound was prepared in the same manner as in Comparative Example 16, except that the amount of the electrophilic compound used in the reaction was reduced as shown in the table below by adjusting the flow velocity of the 1,10-phenanthroline solution C5.

### [Examples 54 and 55]

A desired electrophilic substituted aromatic compound was prepared in the same manner as in Comparative Example 16, except that the moisture content of each solution to be allowed to flow in the flow channel was adjusted and the amount of the electrophilic compound used in the reaction was prepared as shown in the table below by adjusting the flow velocity of the 1,10-phenanthroline solution C5.

The results are shown in the table below.

**[Table 15]**

| | Moisture content (ppm) | Organometallic compound (molar equivalent) | Electrophilic compound (molar equivalent) | Nucleophilic compound (molar equivalent) | LC production rate (%) |
|---|---|---|---|---|---|
| Comparative Example 16 | 225 | 1.0 | 1.0 | 1.0 | 38 |
| Comparative Example 17 | 225 | 1.0 | 0.8 | 1.0 | 13 |
| Example 54 | 31 | 1.0 | 1.0 | 1.0 | 50 |
| Example 55 | 31 | 1.0 | 0.8 | 1.0 | 31 |

### [Comparative Example 18]

A desired electrophilic substituted aromatic compound (desired compound) was prepared using the flow-type reaction system 300 having the configuration shown in FIG. 5. The desired electrophilic substituted aromatic compound was prepared under the following reaction conditions, in which the moisture content of each solution to be allowed to flow in the flow channel was adjusted. Specific reaction conditions are as follows.

### Temperature control:

The temperatures of the flow channels 11 and 12, the joining portion J9, and the reaction tube F9 were set to 0°C.

The temperatures of the flow channel 13, the joining portion J10, and the reaction tube F10 were set to 0°C.

The temperatures of the flow channels 14 and 15, the joining portion J11, and the reaction tube F11 were set to 0°C.

The temperatures of the joining portion J12 and the reaction tube F12 were set to 30°C.

The temperatures of the flow channel 16, the joining portion J13, and the reaction tube F13 were set to 5°C.

### Flow channel 11, flow channel 12, flow channel 13, flow channel 14, flow channel 15, flow channel 16:

An SUS316 tube having an outer diameter of 1/16 inches, an inner diameter of 1.0 mm, and a length of 50 cm was used.

### Reaction tube F9:

An SUS316 tube having an outer diameter of 1/16 inches, an inner diameter of 1.0 mm, and a length of 1.5 m was used.

### Reaction tube F10:

An SUS316 tube having an outer diameter of 1/16 inches, an inner diameter of 1.0 mm, and a length of 1.0 m was used.

### Reaction tube F11:

An SUS316 tube having an outer diameter of 1/16 inches, an inner diameter of 1.0 mm, and a length of 1.5 m was used.

### Reaction tube F12:

An SUS316 tube having an outer diameter of 1/16 inches, an inner diameter of 1.0 mm, and a length of 2.0 m was used.

### Reaction tube F13:

A PTFE tube having an outer diameter of 1/8 inches, an inner diameter of 1.6 mm, and a length of 1.5 m was used.

### Joining portion J9:

A T-shaped connector having an inner diameter of 1.0 mm was used.

The flow channel 11 and the flow channel 12 were respectively connected to two connection ports facing each other among three connection ports of the T-shaped connector. The remaining connection port was used as a discharge port for discharging the liquid.

### Joining portion J10:

A T-shaped connector having an inner diameter of 1.0 mm was used. The reaction tube F9 and the flow channel 13 were respectively connected to two connection ports facing each other among three connection ports of the T-shaped connector. The remaining connection port was used as a discharge port for discharging the liquid.

### Joining portion J11:

A T-shaped connector having an inner diameter of 1.0 mm was used. The flow channel 14 and the flow channel 15 were respectively connected to two connection ports facing each other among three connection ports of the T-shaped connector. The remaining connection port was used as a discharge port for discharging the liquid.

### Joining portion J12:

A T-shaped connector having an inner diameter of 1.0 mm was used. The reaction tube F10 and the reaction tube F11 were respectively connected to two connection ports facing each other among three connection ports of the T-shaped connector. The remaining connection port was used as a discharge port for discharging the liquid.

### Joining portion J13:

A T-shaped connector having an inner diameter of 2.3 mm was used. The reaction tube F12 and the flow channel 16 were respectively connected to two connection ports facing each other among three connection ports of the T-shaped connector. The remaining connection port was used as a discharge port for discharging the liquid.

### Liquid A3 containing halogenated aromatic compound in organic solvent:

A solution obtained by dissolving bromobenzene in THF (bromobenzene solution, bromobenzene concentration: 0.32 M) was prepared.

### Liquid A4 containing halogenated aromatic compound in organic solvent:

A solution obtained by dissolving bromobenzene in THF (bromobenzene solution, bromobenzene concentration: 0.32 M) was prepared.

### Liquid B4 containing organometallic compound in organic solvent:

n-BuLi (n-butyl lithium solution B4, n-BuLi concentration: 1.6 M, n-hexane solution) manufactured by Kanto Chemical Co., Inc. was used.

### Liquid B5 containing organometallic compound in organic solvent:

n-BuLi (n-butyl lithium solution B5, n-BuLi concentration: 1.6 M, n-hexane solution) manufactured by Kanto Chemical Co., Inc. was used.

### Liquid C4 containing electrophilic compound in organic solvent:

A solution obtained by dissolving terephthalaldehyde in THF (terephthalaldehyde solution C4, terephthalaldehyde concentration: 0.37 M) was prepared.

### Liquid feeding conditions:

Bromobenzene solution A3: 6.0 mL/min
Bromobenzene solution A4: 6.0 mL/min
n-Butyl lithium solution B4: 1.2 mL/min
n-Butyl lithium solution B5: 1.2 mL/min
Terephthalaldehyde solution C4: 5.1 mL/min
Water (X): 1.7 mL/min

### [Comparative Example 19]

A desired electrophilic substituted aromatic compound was prepared in the same manner as in Comparative Example 18, except that the amount of the electrophilic compound used in the reaction was reduced as shown in the table below by adjusting the flow velocity of the terephthalaldehyde solution C4.

### [Examples 56 and 57]

A desired electrophilic substituted aromatic compound was prepared in the same manner as in Comparative Example 18, except that the moisture content of each solution to be allowed to flow in the flow channel was adjusted and the amount of the electrophilic compound used in the reaction was prepared as shown in the table below by adjusting the flow velocity of the terephthalaldehyde solution C4.

The results are shown in the table below. The flow channels shown in the table below indicate flow channels into which each compound (solution) was introduced.

**[Table 16]**

| | Moisture content (ppm) | Halogenated aromatic compound (molar equivalent) [flow channel 11] | Organometallic compound (molar equivalent) [flow channel 12] | Electrophilic compound (molar equivalent) [flow channel 13] | Halogenated aromatic compound (molar equivalent) [flow channel 14] | Organometallic compound (molar equivalent) [flow channel 15] | GC production rate (%) |
|---|---|---|---|---|---|---|---|
| Comparative Example 18 | 312 | 1.0 | 1.0 | 1.0 | 1.0 | 1.0 | 74 |
| Comparative Example 19 | 312 | 1.0 | 1.0 | 0.8 | 1.0 | 1.0 | 52 |
| Example 56 | 22 | 1.0 | 1.0 | 1.0 | 1.0 | 1.0 | 81 |
| Example 57 | 22 | 1.0 | 1.0 | 0.8 | 1.0 | 1.0 | 57 |

### [Comparative Example 20]

A desired electrophilic substituted aromatic compound (desired compound) was prepared using the flow-type reaction system 100 having the configuration shown in FIG. 1. The desired electrophilic substituted aromatic compound was prepared under the following reaction conditions, in which the moisture content of each solution to be allowed to flow in the flow channel was adjusted. Specific reaction conditions are as follows.

### Temperature control:

The temperatures of the flow channels 1 and 2, the joining portion J1, and the reaction tube F1 were set to 0°C.

The temperatures of the flow channel 3, the joining portion J2, and the reaction tube F2 were set to 0°C.

The temperatures of the flow channel 4, the joining portion J3, and the reaction tube F3 were set to -20°C.

The temperatures of the flow channel 5, the joining portion J4, and the reaction tube F4 were set to 5°C.

The temperatures of the flow channel 6, the joining portion J5, and the reaction tube F5 were set to 5°C.

### Flow channels 1 to 6:

In all of the flow channels 1 to 6, an SUS316 tube having an outer diameter of 1/16 inches, an inner diameter of 1.0 mm, and a length of 50 cm was used.

### Reaction tubes F1 to F4:

In all of the reaction tubes F1 to F4, an SUS316 tube having an outer diameter of 1/16 inches and an inner diameter of 1.0 mm was used. The length of each flow channel was as follows.
Reaction tube F1: 0.5 m
Reaction tube F2: 2.0 m
Reaction tube F3: 2.0 m
Reaction tube F4: 1.5 m

### Reaction tube F5:

A PTFE tube having an outer diameter of 1/8 inches, an inner diameter of 1.6 mm, and a length of 1.5 m was used.

### Joining portion J1:

A T-shaped connector having an inner diameter of 1.0 mm was used. The flow channel 1 and the flow channel 2 were respectively connected to two connection ports facing each other among three connection ports of the T-shaped connector. The remaining connection port was used as a discharge port for discharging the liquid.

### Joining portion J2:

A T-shaped connector having an inner diameter of 1.0 mm was used. The reaction tube F1 and the flow channel 3 were respectively connected to two connection ports facing each other among three connection ports of the T-shaped connector. The remaining connection port was used as a discharge port for discharging the liquid.

### Joining portion J3:

A T-shaped connector having an inner diameter of 1.0 mm was used. The reaction tube F2 and the flow channel 4 were respectively connected to two connection ports facing each other among three connection ports of the T-shaped connector. The remaining connection port was used as a discharge port for discharging the liquid.

### Joining portion J4:

A T-shaped connector having an inner diameter of 1.0 mm was used. The reaction tube F3 and the flow channel 5 were respectively connected to two connection ports facing each other among three connection ports of the T-shaped connector. The remaining connection port was used as a discharge port for discharging the liquid.

### Joining portion J5:

A T-shaped connector having an inner diameter of 2.3 mm was used. The reaction tube F4 and the flow channel 6 were respectively connected to two connection ports facing each other among three connection ports of the T-shaped connector. The remaining connection port was used as a discharge port for discharging the liquid.

### Liquid A1 containing dihalogenated aromatic compound in organic solvent:

A solution obtained by dissolving 2.7-dibromo-9.9-dimethylfluorene in THF (2.7-dibromo-9.9-dimethylfluorene solution, 2.7-dibromo-9.9-dimethylfluorene concentration: 0.28 M) was prepared.

### Liquid B1 containing organometallic compound in organic solvent:

n-BuLi (n-butyl lithium solution B1, n-BuLi concentration: 1.6 M, n-hexane solution) manufactured by Kanto Chemical Co., Inc. was used.

### Liquid B2 containing organometallic compound in organic solvent:

n-BuLi (n-butyl lithium solution B2, n-BuLi concentration: 1.6 M, n-hexane solution) manufactured by Kanto Chemical Co., Inc. was used.

### Liquid C1 containing electrophilic compound in organic solvent:

A solution obtained by dissolving 2-isopropoxy-4,4,5,5-tetramethyl-1,3,2-dioxaborolane in THF (2-isopropoxy-4,4,5,5-tetramethyl-1,3,2-dioxaborolane solution C1, 2-isopropoxy-4,4,5,5-tetramethyl-1,3,2-dioxaborolane concentration: 0.27 M) was prepared and used in the first stage reaction.

### Liquid C2 containing electrophilic compound in organic solvent:

A solution obtained by dissolving 2-isopropoxy-4,4,5,5-tetramethyl-1,3,2-dioxaborolane in THF (2-isopropoxy-4,4,5,5-tetramethyl-1,3,2-dioxaborolane solution C1, 2-isopropoxy-4,4,5,5-tetramethyl-1,3,2-dioxaborolane concentration: 0.27 M) was prepared and used in the second stage reaction.

### Liquid feeding conditions:

2.7-Dibromo-9.9-dimethylfluorene solution A1: 5.7 mL/min
n-Butyl lithium solution B1: 1.0 mL/min
n-Butyl lithium solution B2: 1.0 mL/min
2-Isopropoxy-4,4,5,5-tetramethyl-1,3,2-dioxaborolane solution C1: 6.0 mL/min
2-Isopropoxy-4,4,5,5-tetramethyl-1,3,2-dioxaborolane solution C2: 7.2 mL/min
Water (X): 1.5 mL/min

### [Comparative Example 21]

A desired electrophilic substituted aromatic compound was prepared in the same manner as in Comparative Example 20, except that the amount of the electrophilic compound used in the reaction was reduced as shown in the table below by adjusting the flow velocity of the 2-isopropoxy-4,4,5,5-tetramethyl-1,3,2-dioxaborolane solution C2.

### [Examples 58 and 59]

A desired electrophilic substituted aromatic compound was prepared in the same manner as in Comparative Example 20, except that the moisture content of each solution to be allowed to flow in the flow channel was adjusted, and the amount of the electrophilic compound used in the reaction was prepared as shown in the table below by adjusting the flow velocity of the 2-isopropoxy-4,4,5,5-tetramethyl-1,3,2-dioxaborolane solution C2.

The results are shown in the table below.

**[Table 17]**

| | Moisture content (ppm) | First stage reaction | | Second stage reaction | | NMR production rate (%) |
|---|---|---|---|---|---|---|
| | | Organometallic compound (molar equivalent) | Electrophilic compound (molar equivalent) | Organometallic compound (molar equivalent) | Electrophilic compound (molar equivalent) | |
| Comparative Example 20 | 308 | 1.0 | 1.0 | 1.0 | 1.2 | 70 |
| Comparative Example 21 | 308 | 1.0 | 1.0 | 1.0 | 1.0 | 61 |
| Example 58 | 23 | 1.0 | 1.0 | 1.0 | 1.2 | 74 |
| Example 59 | 23 | 1.0 | 1.0 | 1.0 | 1.0 | 67 |

### [Comparative Example 22]

A desired electrophilic substituted aromatic compound (desired compound) was prepared using the flow-type reaction system 100 having the configuration shown in FIG. 1. The desired electrophilic substituted aromatic compound was prepared under the following reaction conditions, in which the moisture content of each solution to be allowed to flow in the flow channel was adjusted. Specific reaction conditions are as follows.

### Temperature control:

The temperatures of the flow channels 1 and 2, the joining portion J1, and the reaction tube F1 were set to 0°C.

The temperatures of the flow channel 3, the joining portion J2, and the reaction tube F2 were set to 0°C.

The temperatures of the flow channel 4, the joining portion J3, and the reaction tube F3 were set to -20°C.

The temperatures of the flow channel 5, the joining portion J4, and the reaction tube F4 were set to 5°C.

The temperatures of the flow channel 6, the joining portion J5, and the reaction tube F5 were set to 5°C.

### Flow channels 1 to 6:

In all of the flow channels 1 to 6, an SUS316 tube having an outer diameter of 1/16 inches, an inner diameter of 1.0 mm, and a length of 50 cm was used.

### Reaction tubes F1 to F4:

In all of the reaction tubes F1 to F4, an SUS316 tube having an outer diameter of 1/16 inches and an inner diameter of 1.0 mm was used. The length of each flow channel was as follows.
Reaction tube F1: 0.5 m
Reaction tube F2: 2.0 m
Reaction tube F3: 2.0 m
Reaction tube F4: 1.5 m

### Reaction tube F5:

A PTFE tube having an outer diameter of 1/8 inches, an inner diameter of 1.6 mm, and a length of 1.5 m was used.

### Joining portion J1:

A T-shaped connector having an inner diameter of 1.0 mm was used. The flow channel 1 and the flow channel 2 were respectively connected to two connection ports facing each other among three connection ports of the T-shaped connector. The remaining connection port was used as a discharge port for discharging the liquid.

### Joining portion J2:

A T-shaped connector having an inner diameter of 1.0 mm was used. The reaction tube F1 and the flow channel 3 were respectively connected to two connection ports facing each other among three connection ports of the T-shaped connector. The remaining connection port was used as a discharge port for discharging the liquid.

### Joining portion J3:

A T-shaped connector having an inner diameter of 1.0 mm was used. The reaction tube F2 and the flow channel 4 were respectively connected to two connection ports facing each other among three connection ports of the T-shaped connector. The remaining connection port was used as a discharge port for discharging the liquid.

### Joining portion J4:

A T-shaped connector having an inner diameter of 1.0 mm was used. The reaction tube F3 and the flow channel 5 were respectively connected to two connection ports facing each other among three connection ports of the T-shaped connector. The remaining connection port was used as a discharge port for discharging the liquid.

### Joining portion J5:

A T-shaped connector having an inner diameter of 2.3 mm was used. The reaction tube F4 and the flow channel 6 were respectively connected to two connection ports facing each other among three connection ports of the T-shaped connector. The remaining connection port was used as a discharge port for discharging the liquid.

### Liquid A1 containing dihalogenated aromatic compound in organic solvent:

A solution obtained by dissolving 3.6-dibromo-9-phenylcarbazole in THF (3.6-dibromo-9-phenylcarbazole solution, 3.6-dibromo-9-phenylcarbazole concentration: 0.25 M) was prepared.

### Liquid B1 containing organometallic compound in organic solvent:

n-BuLi (n-butyl lithium solution B1, n-BuLi concentration: 1.6 M, n-hexane solution) manufactured by Kanto Chemical Co., Inc. was used.

### Liquid B2 containing organometallic compound in organic solvent:

n-BuLi (n-butyl lithium solution B2, n-BuLi concentration: 1.6 M, n-hexane solution) manufactured by Kanto Chemical Co., Inc. was used.

### Liquid C1 containing electrophilic compound in organic solvent:

A solution obtained by dissolving 2-isopropoxy-4,4,5,5-tetramethyl-1,3,2-dioxaborolane in THF (2-isopropoxy-4,4,5,5-tetramethyl-1,3,2-dioxaborolane solution C1, 2-isopropoxy-4,4,5,5-tetramethyl-1,3,2-dioxaborolane concentration: 0.27 M) was prepared and used in the first stage reaction.

### Liquid C2 containing electrophilic compound in organic solvent:

A solution obtained by dissolving 2-isopropoxy-4,4,5,5-tetramethyl-1,3,2-dioxaborolane in THF (2-isopropoxy-4,4,5,5-tetramethyl-1,3,2-dioxaborolane solution C1, 2-isopropoxy-4,4,5,5-tetramethyl-1,3,2-dioxaborolane concentration: 0.27 M) was prepared and used in the second stage reaction.

### Liquid feeding conditions:

3.6-Dibromo-9-phenylcarbazole solution A1: 6.5 mL/min
n-Butyl lithium solution B1: 1.0 mL/min
n-Butyl lithium solution B2: 1.0 mL/min
2-Isopropoxy-4,4,5,5-tetramethyl-1,3,2-dioxaborolane solution C1: 6.0 mL/min
2-Isopropoxy-4,4,5,5-tetramethyl-1,3,2-dioxaborolane solution C2: 7.2 mL/min
Water (X): 1.5 mL/min

### [Comparative Example 23]

A desired electrophilic substituted aromatic compound was prepared in the same manner as in Comparative Example 22, except that the amount of the electrophilic compound used in the reaction was reduced as shown in the table below by adjusting the flow velocity of the 2-isopropoxy-4,4,5,5-tetramethyl-1,3,2-dioxaborolane solution C2.

### [Examples 60 and 61]

A desired electrophilic substituted aromatic compound was prepared in the same manner as in Comparative Example 22, except that the moisture content of each solution to be allowed to flow in the flow channel was adjusted, and the amount of the electrophilic compound used in the reaction was prepared as shown in the table below by adjusting the flow velocity of the 2-isopropoxy-4,4,5,5-tetramethyl-1,3,2-dioxaborolane solution C2.

The results are shown in the table below.

**[Table 18]**

| | Moisture content (ppm) | First stage reaction | | Second stage reaction | | NMR production rate (%) |
|---|---|---|---|---|---|---|
| | | Organometallic compound (molar equivalent) | Electrophilic compound (molar equivalent) | Organometallic compound (molar equivalent) | Electrophilic compound (molar equivalent) | |
| Comparative Example 22 | 308 | 1.0 | 1.0 | 1.0 | 1.2 | 82 |
| Comparative Example 23 | 308 | 1.0 | 1.0 | 1.0 | 1.0 | 77 |
| Example 60 | 36 | 1.0 | 1.0 | 1.0 | 1.2 | 89 |
| Example 61 | 36 | 1.0 | 1.0 | 1.0 | 1.0 | 84 |

Although the present invention has been described in conjunction with embodiments thereof, it is not intended to limit the present invention to any of the details of the above description unless specifically stated otherwise, but it is believed that the present invention should be construed broadly without departing from the spirit and scope of the invention as set forth in the claims appended hereto.

This application claims priority based on JP2023-106114 filed on June 28, 2023, the entire contents of which are incorporated herein by reference.

### Explanation of References

100, 200, 300, 400: flow-type reaction system
1 to 21: flow channel (raw material supply flow channel)
J1 to J17: joining portion
F1 to F17: reaction tube (reaction flow channel)
A1: liquid containing halogenated aromatic compound in organic solvent (dihalogenated aromatic compound solution)
A2 to A5: liquid containing halogenated aromatic compound in organic solvent (monohalogenated aromatic compound solution)
B1 to B6: liquid containing organometallic compound in organic solvent (organometallic compound solution)
C1 to C5: liquid containing electrophilic compound in organic solvent (electrophilic compound solution)
D: liquid containing nucleophilic compound in organic solvent (nucleophilic compound solution)
X: Quenching agent

## Claims

1. A method for manufacturing an electrophilic substituted aromatic compound, comprising:
continuously carrying out, by a flow-type reaction, a reaction in which a halogenated aromatic compound is reacted with an organometallic compound to produce a metallated aromatic compound, and the metallated aromatic compound is reacted with an electrophilic compound to produce an electrophilic substituted aromatic compound,
wherein the method includes allowing a liquid containing the halogenated aromatic compound in an organic solvent, a liquid containing the organometallic compound in an organic solvent, and a liquid containing the electrophilic compound in an organic solvent to flow in a flow channel, with each liquid having a moisture content of 200 ppm or less.

2. The method for manufacturing an electrophilic substituted aromatic compound according to claim 1,
wherein, in a case where the flow-type reaction is a one-stage reaction system, the electrophilic compound is used in an amount of 0.80 molar equivalents or more and less than 1.20 molar equivalents with respect to the halogenated aromatic compound, and
in a case where the flow-type reaction is a multi-stage reaction system, the electrophilic compound is supplied to a final stage reaction in an amount of 0.80 molar equivalents or more and less than 2.00 molar equivalents with respect to the halogenated aromatic compound.

3. The method for manufacturing an electrophilic substituted aromatic compound according to claim 1,
wherein, in a case where the flow-type reaction is a one-stage reaction system, the electrophilic compound is used in an amount of 0.85 molar equivalents or more and 1.05 molar equivalents or less with respect to the halogenated aromatic compound, and
in a case where the flow-type reaction is a multi-stage reaction system, the electrophilic compound is supplied to a final stage reaction in an amount of 0.80 molar equivalents or more and 1.50 molar equivalents or less with respect to the halogenated aromatic compound.

4. The method for manufacturing an electrophilic substituted aromatic compound according to claim 1,
wherein the flow-type reaction is a multi-stage reaction system, and the electrophilic compound is supplied to a final stage reaction in an amount of 0.80 molar equivalents or more and 1.20 molar equivalents or less with respect to the halogenated aromatic compound.

5. The method for manufacturing an electrophilic substituted aromatic compound according to any one of claims 1 to 4, further comprising:
preparing a liquid A1 containing the halogenated aromatic compound in an organic solvent, a liquid B1 and a liquid B2 each containing the organometallic compound in an organic solvent, and a liquid C1 and a liquid C2 each containing the electrophilic compound in an organic solvent;
introducing the liquids A1, B1, B2, C1, and C2 into different flow channels to allow each liquid to flow in each flow channel; and
allowing the liquid A1 and the liquid B1 to join together to form a joined liquid M1, in which a metallated halogeno-aromatic compound is produced in the joined liquid M1 while the joined liquid M1 flows downstream in a reaction flow channel F1; allowing the joined liquid M1 and the liquid C1 to join together to form a joined liquid M2, in which an electrophilic monosubstituted halogeno-aromatic compound is produced in the joined liquid M2 while the joined liquid M2 flows downstream in a reaction flow channel F2; allowing the joined liquid M2 and the liquid B2 to join together to form a joined liquid M3, in which a metallated electrophilic monosubstituted aromatic compound is produced in the joined liquid M3 while the joined liquid M3 flows downstream in a reaction flow channel F3; and allowing the joined liquid M3 and the liquid C2 to join together to form a joined liquid M4, in which an electrophilic disubstituted aromatic compound is produced in the joined liquid M4 while the joined liquid M4 flows downstream in a reaction flow channel F4.

6. The method for manufacturing an electrophilic substituted aromatic compound according to any one of claims 1 to 3, further comprising:
preparing a liquid A2 containing the halogenated aromatic compound in an organic solvent, a liquid B3 containing the organometallic compound in an organic solvent, and a liquid C3 containing the electrophilic compound in an organic solvent;
introducing the liquids A2, B3, and C3 into different flow channels to allow each liquid to flow in each flow channel; and
allowing the liquid A2 and the liquid B3 to join together to form a joined liquid M6, in which a metallated aromatic compound is produced in the joined liquid M6 while the joined liquid M6 flows downstream in a reaction flow channel F6; and allowing the joined liquid M6 and the liquid C3 to join together to form a joined liquid M7, in which an electrophilic monosubstituted aromatic compound is produced in the joined liquid M7 while the joined liquid M7 flows downstream in a reaction flow channel F7.

7. The method for manufacturing an electrophilic substituted aromatic compound according to any one of claims 1 to 3, further comprising:
preparing a liquid A3 and a liquid A4 each containing the halogenated aromatic compound in an organic solvent, a liquid B4 and a liquid B5 each containing the organometallic compound in an organic solvent, and a liquid C4 containing the electrophilic compound in an organic solvent;
introducing the liquids A3, A4, B4, B5, and C4 into different flow channels to allow each liquid to flow in each flow channel; and
allowing the liquid A3 and the liquid B4 to join together to form a joined liquid M9, in which a metallated aromatic compound is produced in the joined liquid M9 while the joined liquid M9 flows downstream in a reaction flow channel F9; allowing the liquid A4 and the liquid B5 to join together to form a joined liquid M11, in which a metallated aromatic compound is produced in the joined liquid M11 while the joined liquid M11 flows downstream in a reaction flow channel F11; allowing the joined liquid M9 and the liquid C4 to join together to form a joined liquid M10, in which an electrophilic monosubstituted aromatic compound (i) functioning as an electrophilic compound is produced in the joined liquid M10 while the joined liquid M10 flows downstream in a reaction flow channel F10; and allowing the joined liquid M11 and the joined liquid M10 to join together to form a joined liquid M12, in which the metallated aromatic compound in the joined liquid M11 reacts with the electrophilic monosubstituted aromatic compound (i) in the joined liquid M10 to produce an electrophilic monosubstituted aromatic compound in the joined liquid M12 while the joined liquid M12 flows downstream in a reaction flow channel F12.

8. The method for manufacturing an electrophilic substituted aromatic compound according to any one of claims 1 to 3, further comprising:
preparing a liquid A5 containing the halogenated aromatic compound in an organic solvent, a liquid B6 containing the organometallic compound in an organic solvent, a liquid C5 containing the electrophilic compound in an organic solvent, and a liquid D containing a nucleophilic compound in an organic solvent;
introducing the liquids A5, B6, C5, and D into different flow channels to allow each liquid to flow in each flow channel; and
allowing the liquid A5 and the liquid B6 to join together to form a joined liquid M14, in which a metallated aromatic compound is produced in the joined liquid M14 while the joined liquid M14 flows downstream in a reaction flow channel F14; allowing the joined liquid M14 and the liquid C5 to join together to form a joined liquid M15, in which an electrophilic monosubstituted aromatic compound is produced in the joined liquid M15 while the joined liquid M15 flows downstream in a reaction flow channel F15; and allowing the joined liquid M15 and the liquid D to join together to form a joined liquid M16, in which an electrophilic monosubstituted/nucleophilic substituted aromatic compound is produced in the joined liquid M16 while the joined liquid M16 flows downstream in a reaction flow channel F 16.

9. The method for manufacturing an electrophilic substituted aromatic compound according to any one of claims 1 to 4,
wherein all of moisture contents of the liquid containing the halogenated aromatic compound in an organic solvent, the liquid containing the organometallic compound in an organic solvent, and the liquid containing the electrophilic compound in an organic solvent, each of which is allowed to flow in the flow channel, are controlled to be 100 ppm or less.
